# EUROPEAN PATENT SPECIFICATION

(11) **EP 3 496 741 B1**
(45) Date of publication and mention of the grant of the patent: **05.10.2022**
(21) Application number: 17841991.7
(22) Date of filing: 15.08.2017
(51) Int. Cl.: A61K 38/17, A61K 48/00, A61P 3/10, C12N 15/86, C07K 14/00

(54) **NOVEL THERAPY TO ACHIEVE GLYCEMIC CONTROL**
NEUARTIGE THERAPIE ZUR ERZIELUNG VON GLYKÄMISCHER KONTROLLE
NOUVEAU TRAITEMENT PERMETTANT D'OBTENIR UNE RÉGULATION GLYCÉMIQUE

(30) Priority: 15.08.2016 US 201662375427 P; 01.09.2016 US 201662382726 P
(43) Date of publication of application: 19.06.2019
(73) Proprietor: Beth Israel Deaconess Medical Center, Inc., Boston, MA 02215 (US)
(72) Inventor: FERRAN, Christiane, Watertown MA 02472 (US); DA SILVA, Cleide, Brighton MA 02135 (US); MELE, Alessandra, Brookline MA 02245 (US)
(74) Representative: HGF
(86) International application number: PCT/US2017/046938
(87) International publication number: WO 2018/035121

(56) References cited:
- WO-A1-2016/034611
- CN-A- 104 043 108
- US-A1- 2003 171 253
- Yu Ly ET AL: "Direct transfer of A20 gene into pancreas protected mice from streptozotocin-induced diabetes 1", Acta Pharmacol. Sin., 1 June 2004 (2004-06-01), pages 721-726, XP055678410, Retrieved from the Internet: URL:http://www.chinaphar.com/article/view/ 8206/8811 [retrieved on 2020-03-20]
- ZHANG MIN ET AL: "Roles of A20 in autoimmune diseases", IMMUNOLOGIC RESEARCH, HUMANA PRESS, INC, US, vol. 64, no. 2, 2 July 2015 (2015-07-02), pages 337-344, XP035952404, ISSN: 0257-277X, DOI: 10.1007/S12026-015-8677-6 [retrieved on 2015-07-02]

## Description

### FIELD OF INVENTION

The invention relates to an effective amount of A20 for use in treating a condition selected from the group consisting of hyperglycemia, diabetes, pre-diabetes, insulin resistance and metabolic syndrome, wherein the A20 is delivered to the liver, as well as agents that upregulate A20 expression.

### BACKGROUND

Dysregulation of blood glucose is associated with a range of pervasive and damaging medical conditions, the treatment of which is expensive, inconvenient, and often ineffective. There is a pressing need for treatments that can mitigate, halt, and/or reverse conditions associated with glucose dysregulation, such as diabetes mellitus.

CN104043108 describes mouse models with an A20 gene knockout. Yu Ly et al., 2004 describes delivery of a plasmid to the pancreas to increase expression of A20 in the pancreas.

### SUMMARY

The present invention is defined by the appended claims.

This disclosure is based, in part, on the unexpected discovery that hepatic overexpression of A20 restores glycemic control to treat one or more sign or symptom of hyperglycemia, diabetes, and related conditions. Previously established effects of A20 in the liver related only to its anti-inflammatory, anti-apoptotic, and pro-regenerative functions, but there was no indication that it also improves glucose metabolism.
Additionally, the data indicates that overexpression of A20 in the liver not only positively influences local hepatic glucose metabolism, but also systematically impacts the regulation of glucose metabolism in other organs and tissues. Furthermore, A20 was found to restore glycemic control in an insulin-independent manner, without causing hypoglycemia, even under fasting conditions.

Disclosed herein are methods of treating a metabolic disease or condition including but not limited to hyperglycemia, diabetes, pre-diabetes, insulin resistance and metabolic syndrome in a subject in need thereof. In some embodiments, the method comprises administering A20 to a subject in need thereof in an effective amount to treat the condition. In some embodiments, the condition is insulin-dependent diabetes (Type 1 diabetes), Type 2 diabetes, or gestational diabetes.

In some embodiments, administering A20 comprises administering A20 protein to the subject. In some embodiments, administering A20 comprises A20 gene therapy.

The method comprises administering an agent that upregulates A20 expression in one or more tissues in the subject in an effective amount to treat the condition, wherein the tissue is liver. The method comprises upregulating A20 expression in a tissue of a subject, wherein the tissue is liver. In some embodiments, the agent used to upregulate A20 expression comprises a nucleic acid encoding the gene for A20 in an expression system. In some embodiments, the expression system comprises one or more promoters.

In some embodiments, methods disclosed herein include increasing the expression of endogenous A20 in the subject. In some embodiments, increasing the expression of endogenous A20 in the subject comprises activating one or more endogenous promoters of A20. In some embodiments, increasing the expression of endogenous A20 in the subject comprises editing the genome of the subject. In some embodiments, editing the genome of the subject can be achieved by inserting one or more exogenous promoter(s), enhancer(s) or repressor(s). In some embodiments, editing the genome of the subject can be achieved by deleting or disabling an endogenous mechanism that controls or limits the expression of endogenous A20 in the subject, for instance microRNAs, regulatory long non coding RNA, or anti-sense RNA. In some embodiments, administering the agent restores euglycemia in the subject.

Further disclosed herein are methods of treating insulin-dependent diabetes mellitus in a subject, comprising administering an agent that upregulates A20 in the liver of the subject. In some embodiments, the agent comprises an expression system. In some embodiments, the expression system includes one or more promoters. In some embodiments, the expression system includes a nucleic acid that encodes A20.

In some embodiments of the methods disclosed herein, the expression system is a viral vector. In some embodiments, the viral vector comprises a recombinant AAV vector. In some embodiments, the AAV vector comprises a genome derived from AAV serotype AAV2. In some embodiments, the AAV vector is modified to comprise a capsid with tropism for tissue in the liver. In some embodiments, the AAV vector comprises a capsid protein that is derived from AAV serotype AAV8. In some embodiments, administering A20 restores euglycemia in the subject.

Methods disclosed herein can be used alone or in combination with other therapies or therapeutic agents, for example agents that reduce blood glucose such as insulin and/or oral hypoglycemic agents. For example, the disclosed methods can be used in combination with insulin therapy (e.g., insulin glulisine, insulin lispro, insulin aspart, insulin glargine, insulin detemir insulin isophane), metformin, sulfonylureas (e.g., glyburide, glipizide, glimepiride), meglitinides (e.g., repaglinide or nateglinide), thiazolidinediones (e.g., rosiglitazone, pioglitazone), DPP-4 inhibitors (e.g., sitagliptin, saxagliptin, linagliptin), GLP-1 receptor agonists (e.g., exenatide or liraglutide), and/or SGLT2 inhibitors (e.g., canagliflozin or dapagliflozin).

### BRIEF DESCRIPTION OF THE FIGURES

The following drawings form part of the present specification and are included to further demonstrate certain aspects of the present disclosure, which can be better understood by reference to one or more of these drawings in combination with the detailed description of specific embodiments presented herein.

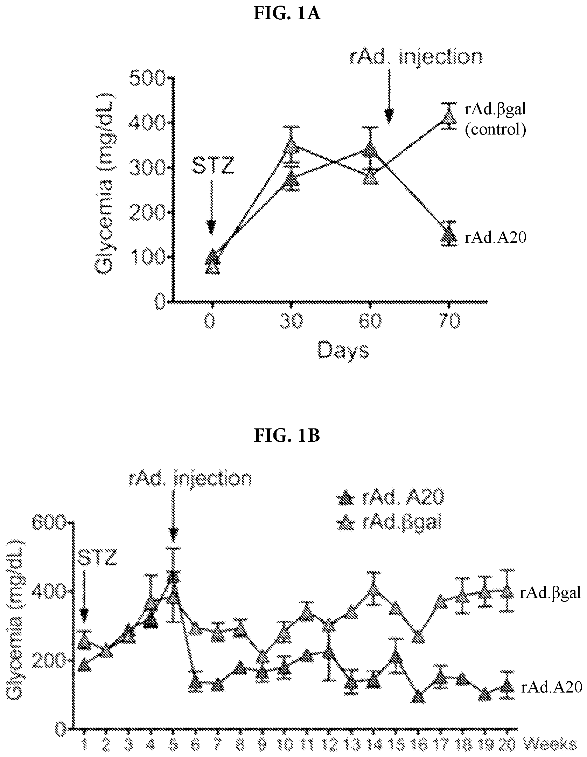

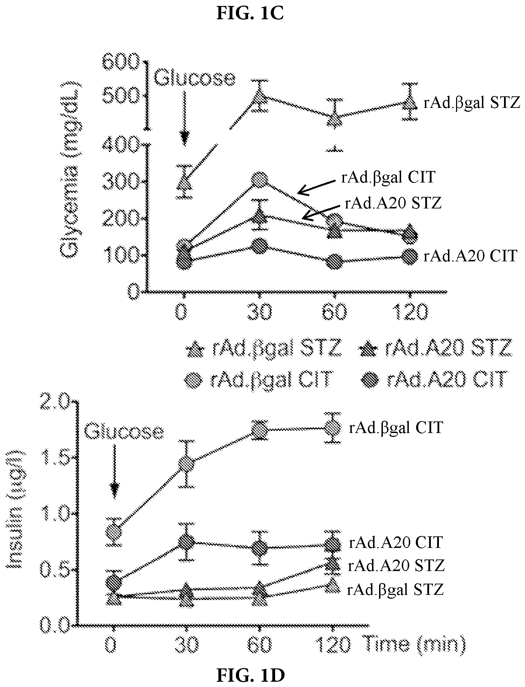

FIGs. 1A-1D show the overexpression of A20 in the liver of diabetic C57BL/6 mice restores euglycemia. FIG. 1A shows the normalization of blood glucose levels within one week of IV injection of 1×10⁹ multiplicity of infection (MOI) of rAd.A20 but not control rAd.βgal in mice that were diabetic for 5-6 weeks (n=5). FIG. 1B shows the fasting euglycemia persisted for >100 days. FIG. 1C shows rAd.A20 STZ mice normalized their GTT curve, whereas it was highly abnormal in rAd.βgal STZ mice. The GTT curve was flat in non-diabetic rAd.A20, i.e. citrate buffer treated mice (CIT), as compared to control rAd.βgal CIT mice. This suggests that A20 improves handling of the glucose load, even in nondiabetic mice (n=3-5). FIG. 1D shows plasma insulin levels, measured by a super sensitive ELISA were at the lowest detection levels in rAd.A20 STZ mice and rAd.βgal STZ mice. As expected, insulin levels increased in non-diabetic mice after the glucose load, but this increase was significantly greater in rAd.βgal CIT vs. rAd.A20 CIT mice (n=3-5). Treatment groups include: rAd.A20 STZ, rAd.βgal STZ, rAd.A20 CIT, and rAd.βgal CIT as indicated in the figure.

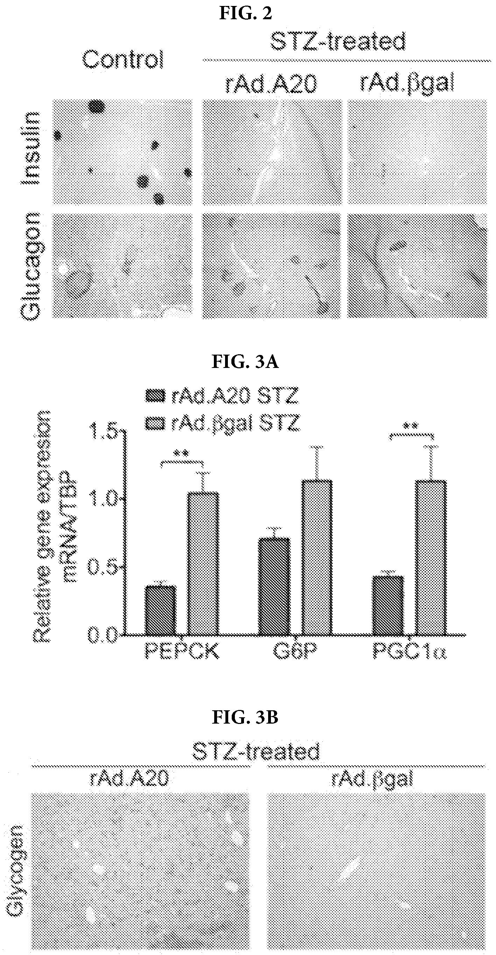

FIG. 2 shows Insulin and glucagon immunostaining in pancreata of control non-diabetic and STZ-diabetic C57BL/6 mice 10 days after IV injection with rAd.A20 or rAd.βgal. A20-treated STZ-diabetic mice had become euglycemic, whereas βgal treated STZ-diabetic mice were still hyperglycemic. A20-treated STZ-diabetic mice that became euglycemic had no evidence of insulin in their pancreas.

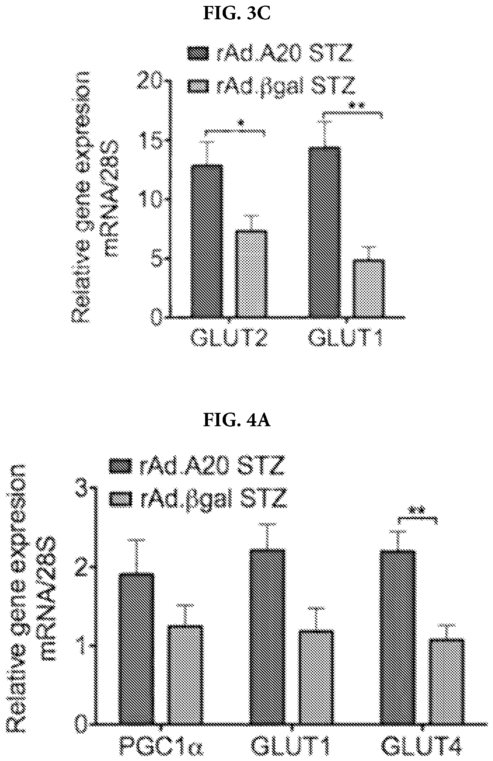

FIGs. 3A-3C show lower hepatic PEPCK, G6P, and PGC1α mRNA levels (FIG. 3A), Higher glycogen levels (n=2) (FIG. 3B), and higher GLUT2 and GLUT1 mRNA levels (FIG. 3C) in livers of rAd.A20 STZ vs. rAd.βgal STZ mice, as determined by qRT-PCR and PAS immunostaining (n=2). Relative mRNA levels were corrected by the house keeping genes TBP or ribosomal 28S.

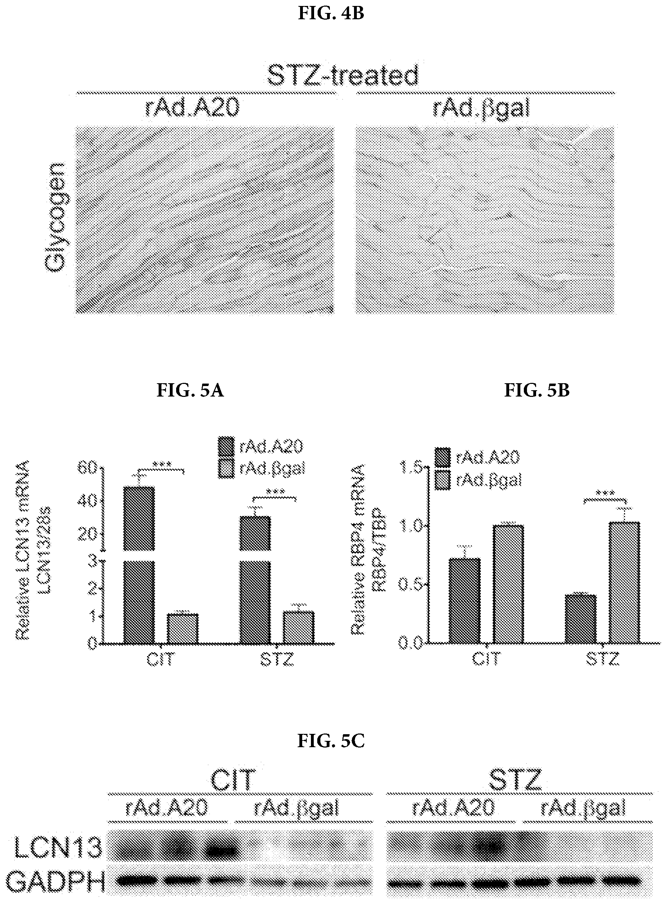

FIGs. 4A-4B show higher GLUT1 GLUT4, and PGC1α mRNA levels (n=3-4) (FIG. 4A) and higher glycogen levels (n=2) (FIG. 4B) in skeletal muscles of rAd.A20 STZ vs. rAd.βgal STZ mice, as determined by qRT-PCR, and PAS glycogen immunostaining. Relative mRNA levels were corrected by the house keeping gene ribosomal 28S. ^{∗∗} <0.01.
FIGs. 5A-5C show higher LCN13 mRNA (FIG. 5A) and protein (FIG. 5C) and lower RBP4 (FIG. 5B) mRNA levels in livers of both diabetic (STZ) and non-diabetic (CIT) rAd.A20 vs. rAd. βgal treated mice. ^{∗∗∗}P<0.001 (n=3-4).

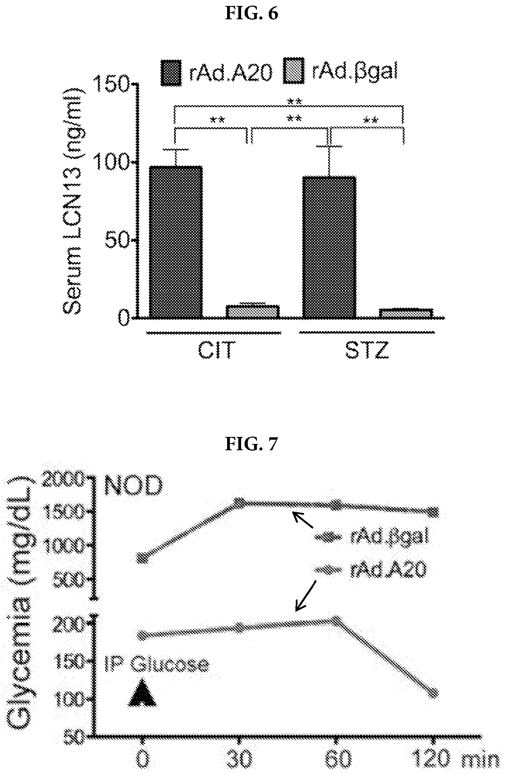

FIG. 6 shows higher LCN13 mRNA and protein levels in livers of diabetic (STZ) and non-diabetic (CIT) rAd.A20 vs. rAd.βgal-treated mice which corresponds with significantly higher (20 fold) circulating LCN13 levels in the sera of these mice as compared to control rAd.βgal-treated mice. (n=5 STZ, 4 CIT) ^{∗∗}P<0.01, ^{∗∗∗}P<0.001. Notably, rAd.A20 STZ mice had normalized their glycemia at the time of serum retrieval, i.e.10 days after rAd. injection.
FIG. 7 shows improved GTT curve in a NOD mouse 8 weeks following two IV injections of rAd.A20 that cured diabetes.

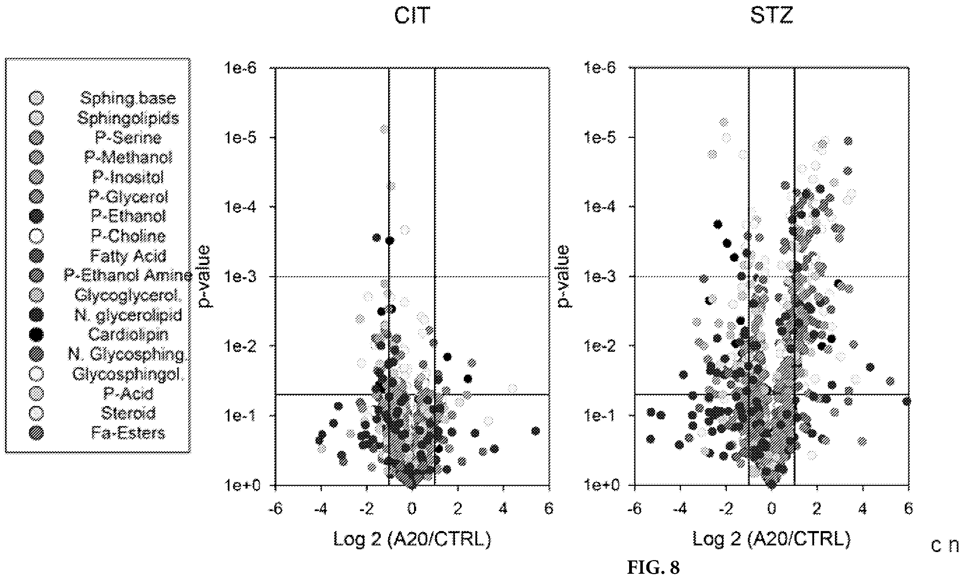

FIG. 8 shows a volcano plot diagram indicating significantly (p value <0.05) changed (+ or - Log 2 fold) lipid species in rAd.A20 vs. rAd.βgal treated livers of non-diabetic (CIT) and diabetic (STZ) mice, 10 days after administration of rAd. Lipid species are coded by class. Notably, the number of lipid species differentially expressed in livers of rAd.A20 vs. rAd.βgal treated mice is much greater in diabetic mice.

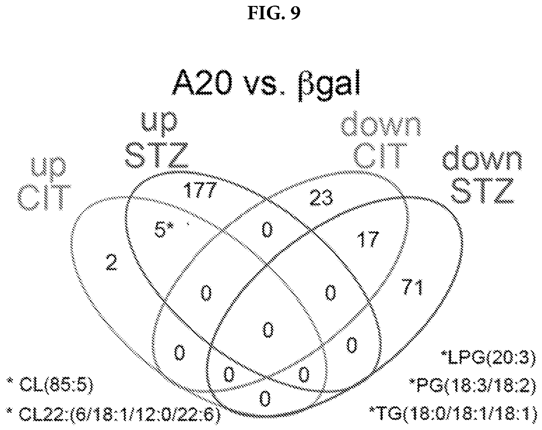

FIG. 9 shows a Venn diagram indicating the number of lipid species significantly up or down-regulated in rAd.A20 vs. rAd.βgal treated livers, highlighting 5 lipids that were significantly increased in both CIT and STZ groups.

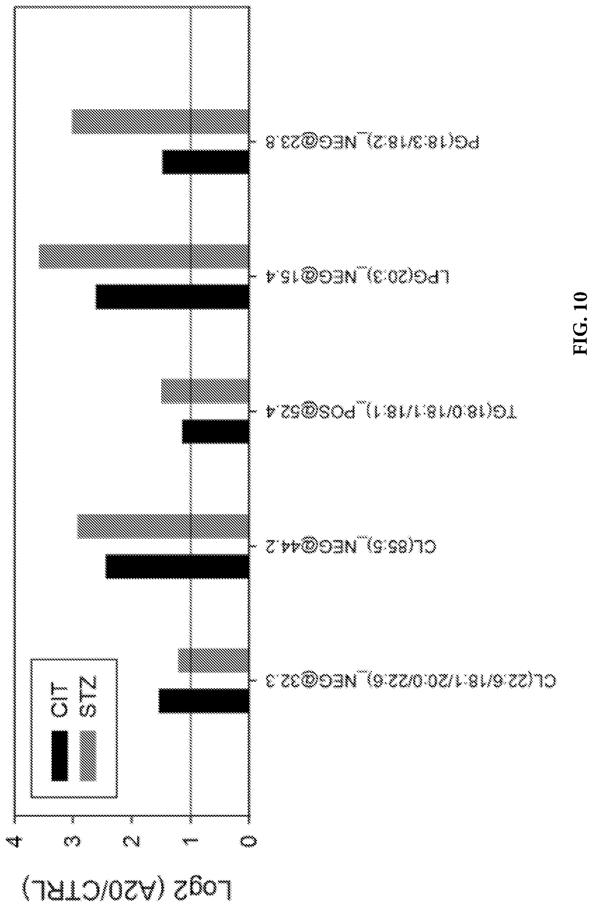

FIG. 10 shows the identity of the five lipids that showed a similar 2 to 10 fold significant increase in the livers of rAd.A20 STZ treated mice, as compared to rAd.βgal treated control (CTRL) mice. The results are represented as log2 value of the group average A20/CTRL ratio for each lipid. A value of 1 indicates a 2-fold increase in the A20 treatment. Four of these lipids were identified by the negative ionization mode, and one was identified by the positive ionization mode. The number of mice/group equaled 4-5 for STZ and 3 for CIT.

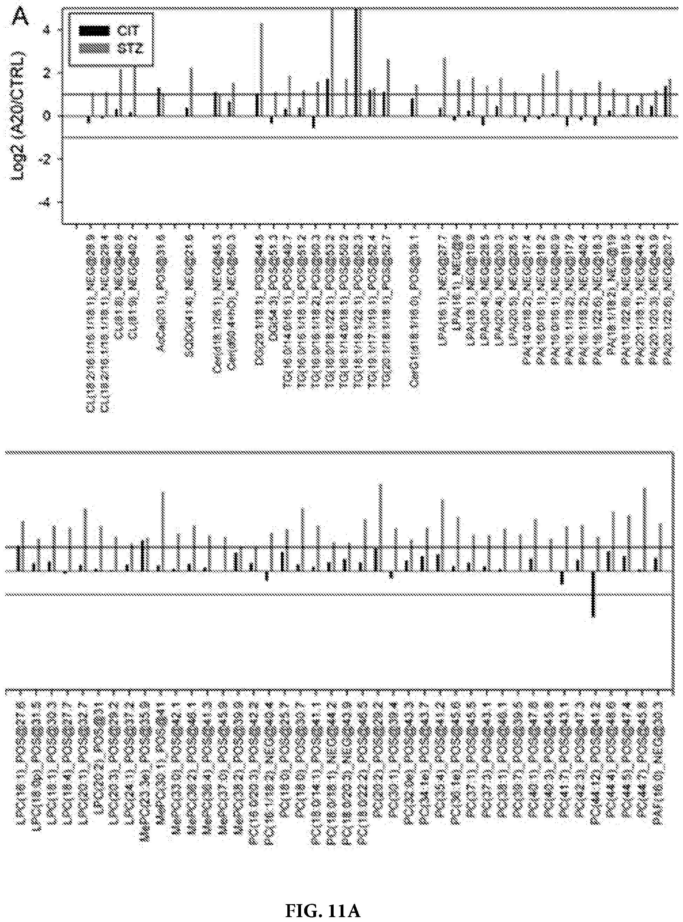

FIGs. 11A to 11B show the identity of the 177 lipids that were significantly and exclusively increased (2 to >10 fold) in the livers of rAd.A20 STZ but not in CIT treated mice, as compared to rAd.βgal treated control (CTRL) mice (FIGs. 11A to 11B). The results are represented as the log2 value of the group average A20/CTRL ratio for each lipid. A value of 1 indicates a 2-fold increase in the A20 treatment. Lipids were annotated by class and identified by either negative or positive ionization mode. The number of mice/groups equaled 4-5 for STZ and 3 for CIT.

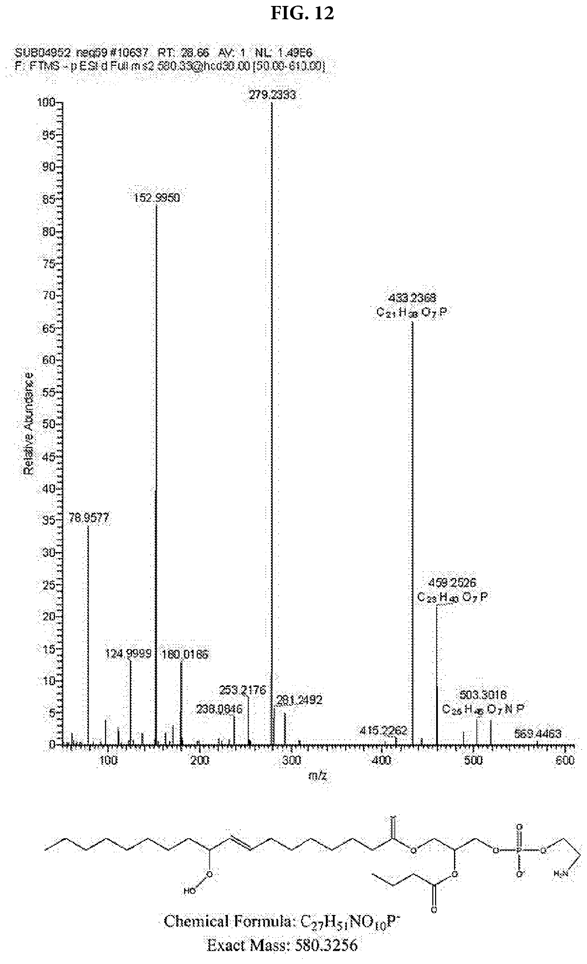

FIG. 12 shows a lipid species that was significantly increased in rAd.A20 STZ livers but not recognized by METLIN search which was tentatively identified based on its accurate mass formula (C27H5101ONP) as a potentially oxidized phosphatidylethalonamine (C18:2/C4).

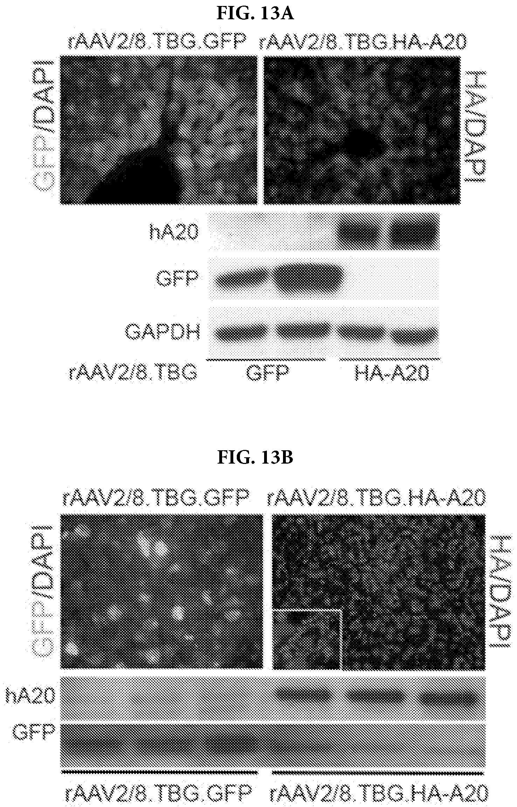

FIGs. 13A-13B show high levels of GFP and A20 as verified by IF and Western blot analysis in mouse livers 3 days after IV injection of AAV 2/8 expressing GFP or A20 and administered IV at 10¹¹ (viral genome) vg/mouse (FIG. 13A), and in swine livers 2 weeks after IV injection of 10¹² vg/Kg of hepatocyte-specific rAAV2/8 expressing GFP and A20, respectively (FIG. 13B). GFP or HA-tagged A20 were expressed under the hepatocyte specific promoter TBG. DAPI stained nuclei in blue.

### DETAILED DESCRIPTION

This disclosure relates to compositions and methods for modifying the concentration of the protein A20 in a subject to treat hyperglycemia, diabetes, pre-diabetes, insulin resistance, metabolic syndrome and related conditions.

A20, is also known as TNF alpha induced protein 3 (TNFAIP3), and OTU domain-containing protein 7C (OTUD7C). The nucleic acid sequence of human A20 (SEQ ID NO: 1, GenBank: M59465.1) encodes a 790 amino acid human A20 protein (SEQ ID NO: 2, GenBank:AAA51550.1).

The invention is not limited in application to a specific vertebrate species or by the use of any specific vertebrate ortholog of the gene or protein encoded by the gene, either homologously (in the species in which the gene or protein itself originates) or heterologously by the expression of the gene of a first species or use of the protein of a first species to effect therapy in a second species. Although it is preferable to use species-specific sequences, the nucleic acid sequence of mouse A20 (SEQ ID NO: 3, GenBank: BC060221.1) that encodes a 775 amino acid mouse A20 protein (SEQ ID NO: 4, GenBank: AAC52153.1) may, for example, be used to effect therapy in a human subject, and vice versa.

A20 has a N-terminal OTU (ovarian tumor) domain and seven repeats of A20-like ZnF (zinc finger) domains. The N-terminal OTU domain of A20 is a deubiquitinase and the ZnF domains (more precisely a region between ZnF 4 and 5) confers E3 ubiquitin ligase activity. A20 ensures optimal responses in cells stimulated by cytokines, such as TNF and IL-1, or pathogen components due, in part, to its ability to negatively regulate inflammatory responses by secondarily regulating NF-KB signaling. Furthermore, A20 exerts anti- or pro-apoptotic and anti-or pro-regenerative functions in a cell-type specific manner. For example, A20 is anti-apoptotic and pro-regenerative in hepatocytes but pro-apoptotic and anti-proliferative in the vascular smooth muscle cells of the intimal layer of the vessel.

A20 encompasses native, wild type, as well as synthetic and recombinant A20. In some embodiments, A20 is an A20 isoform, analog, variant, fragment or functional derivative of A20.

A20 isoforms include versions of A20 with some small differences in their nucleic acid sequence or amino acid sequence, such as, for example, a splice variant or the result of some posttranslational modification.

An A20 analog refers to a compound substantially similar in function to either the native A20 or to a fragment thereof. A20 analogs include, for example, biologically active sequences substantially similar to the A20 sequences and may have substituted, deleted, elongated, replaced, or otherwise modified sequences that possess bioactivity substantially similar to that of A20. For example, an analog of A20 is one which does not have the same sequence as A20 but which is sufficiently homologous to A20 so as to retain the activity of A20. A20 activity assays are known to those of ordinary skill in the art.

An A20 fragment is meant to include any portion of a A20 which provides a segment of A20 which maintains the activity of A20; the term is meant to include A20 fragments which are made from any source, such as, for example, from naturally-occurring sequences, synthetic or chemically-synthesized sequences, and genetically engineered sequences.

An A20 variant is meant to refer to a compound substantially similar in structure and activity either to native A20, or to a fragment thereof.

A functional derivative of A20 is a derivative which possesses an activity that is substantially similar to the activity of A20. By substantially similar is meant activity which is quantitatively different but qualitatively the same. For example, a functional derivative of A20 could contain the same sequence backbone as A20 but also contains other modifications such as, for example, post-translational modifications such as, for example, bound phospholipids, covalently linked carbohydrate, or an added moiety (such as, for example, a sequence that directs the A20 to a cell), depending on the necessity of such modifications. As used herein, the term is also meant to include a chemical derivative of A20. Such derivatives may improve A20's solubility, absorption, biological half-life, or direct it to a cell, etc. The derivatives may also decrease the toxicity of A20, or eliminate or attenuate any undesirable side effect of A20, etc. Chemical moieties capable of mediating such effects are disclosed in Remington's Pharmaceutical Sciences (1980). Procedures for coupling such moieties to a molecule such as A20 are well known in the art. The term functional derivative is intended to include the fragments, variants, analogues, or chemical derivatives of A20.

The present disclosure provides that overexpression of A20 in the liver positively influences local hepatic glucose metabolism, systematically impacts the regulation of glucose metabolism in other organs and tissues, and restores glycemic control in an insulin-independent manner, without causing hypoglycemia, even under fasting conditions.

As used herein, the term "treat" is intended to include prophylaxis, amelioration, alleviation, prevention or cure of a disease or condition, or sign or symptom of a condition, or a predisposition toward the condition, with the purpose to cure, heal, alleviate, relieve, alter, remedy, ameliorate, improve, or affect the condition, the sign or symptom of the condition, or the predisposition toward the condition. Treatment after a condition has started aims to reduce, ameliorate or altogether eliminate the condition, and/or one or more of its associated sign or symptom, or prevent it from becoming worse. Treating a condition does not necessarily require curative results. Treatment of a subject before a condition has started (i.e., prophylactic treatment) aims to reduce the risk of developing the condition and/or lessen its severity if the condition later develops. As used herein, the term "prevent" refers to the prophylactic treatment of a subject who is at risk of developing a condition which treatment results in a decrease in the probability that the subject will develop the condition, or results in an increase in the probability that the condition is less severe than it would have been absent the treatment. Cognate terms related to the word "treat", such as "treating" and "treated" and "treatment" are to be construed in the light of this definition.

Treating can include one or more elements in the process of administering medical care to a subject having a condition in need of care, where medical care comprises diagnosing the likely cause of a condition, determining an appropriate course of action to ameliorate the condition and/or remediate the cause of the condition, executing a course of action with respect to the subject in need of care, monitoring the subject with respect to the course of action, and/or adjusting or terminating the course of action. Treatment may include administering one or more doses of a pharmaceutical, drug, biologic, cell, gene and/or tissue-based therapy, as well as monitoring and follow-up of the subject.

As used herein, the term "hyperglycemia" means an elevated blood glucose level, wherein the fasting blood glucose level (e.g., based on a blood glucose measurement taken in the morning prior to eating or drinking anything likely to elevate blood glucose) is greater than 100 mg/dL, and/or wherein non-fasting blood glucose measurements taken at random or throughout the day show blood glucose level elevated above 125 mg/dL at a frequency that is greater than occasional (e.g., more than 1 in 10 measurements).

As used herein, "diabetes" is defined as a condition characterized by hyperglycemia due to beta cell destruction, sometimes leading to absolute insulin deficiency, and with sequelae of chronic hyperglycemia. Diabetes is a condition characterized by hyperglycemia resulting from variable degrees of insulin resistance, and/or insulin deficiency, and/or insulin dysfunction. This can lead to multi-organ damage, resulting in renal, neurologic, cardiovascular, ophthalmic and other serious complications.

Diabetes includes, for example, Type 1 diabetes (insulin-dependent diabetes mellitus), Type 2 diabetes, gestational diabetes, maturity onset diabetes of the young, Rabson-Meendenhall Syndrome, Donahue Syndrome, diabetic pathophysiology as a result of mitochondrial DNA mutations, diabetes caused by genetic defects in insulin processing or insulin action, such as defective proinsulin conversion, mutations to the insulin gene itself or in the insulin receptor, exocrine defects of the pancreas causing diabetes, diabetes secondary to chronic pancreatitis, pancreatectomy, pancreatic neoplasia, diabetes related to cystic fibrosis, hemochromatosis or fibrocalculous pancreatopathy; endocrinopathies leading to diabetes, acromegaly associated diabetes, diabetes associated with Cushing's syndrome, Down's syndrome, Klinefelter syndrome, and Turner syndrome, hyperthyroidism, pheochromocytoma, glucagonoma, infection with coxsackievirus B or CMV or HCV, lipodystrophy, diabetes resulting from side effects and/or toxicity of drugs including statins, thyroid hormone, glucocorticoids or beta-andregenic agonists, pentamidine, nicotinic acid, didanosine stavudine, zidovudine, indinavir, lopinavir/ritonavir, or diabetes secondary to exposure to toxins such as dioxin.

The term "metabolic syndrome" as used herein refers to a group of commonly co-occurring metabolic risk factors associated with cardiovascular disease and type 2 diabetes mellitus, and obesity. These risk factors include elevated blood pressure, atherogenic dyslipidemia, and insulin resistance. Metabolic syndrome's most commonly accepted diagnostic criteria are derived from the International Diabetes Federation (IDF) Task Force on Epidemiology and Prevention and the American Heart Association/National Heart, Lung, and Blood Institute (AHA/NHLBI), whereby diagnosis requires 3 of the following 5 criteria: (1) triglycerides ≥ 150 mg/dL (1.7 mmol/L) or drug treatment for elevated triglycerides, (2) fasting glucose ≥ 100 mg//dL or drug treatment of elevated glucose, (3) reduced high-density lipoprotein cholesterol or drug treatment for reduced high-density lipoprotein cholesterol (in men, < 40 mg/dL (1.0 mmol/L) or in women, < 50 mg/dL (1.3 mmol/L)), (4) elevated blood pressure, including any of systolic blood pressure ≥ 130 mm Hg, diastolic blood pressure ≥ 85 mm Hg or antihypertensive drug treatment in a subject with a history of hypertension and (5) increased waist circumference, as determined by population- and country-specific thresholds as further defined and refined from time-to-time by IDF and AHA/NHLBI.

As used herein, the term "pre-diabetes" refers to a spectrum of conditions that indicate increased risk of diabetes, and may signal the onset of diabetes, typically characterized by criteria promulgated by the American Diabetes Association, which comprise one or more of: (1) fasting plasma glucose level of between 100 to 125 mg/dL (5.6 to 6.9 mmol/L) also known as impaired fasting glucose; (2) and/or plasma glucose two hours following administration of the 75 gram oral glucose tolerance test of between 140 to 199 mg/dL (7.8 to 11.0 mmol/L) also known as impaired glucose tolerance; (3) and/or glycated hemoglobin (A1C) of between 5.7 to 6.4 % (39 to 46 mmol/mol). For all three tests, risk is actually continuous, extending below the lower limit of the range and becoming disproportionately greater at higher ends of the range.

The term "agent that upregulates A20" as used herein is an agent that raises the physiological level of the protein A20 in one or more tissues. The agent can be any one or more of a small molecule drug, a biologic drug, a cell-based therapy, a nucleic-acid based therapy, including by not limited to a gene therapy, or a tissue-based therapy. The agent may include the A20 protein A20 or any peptide derived from A20 modified to be expressed inside the cells, a nucleic acid encoding for A20, or a substance that causes the production of A20 (either by containing a gene for A20 in a format causing its expression, or by promoting the activity of an endogenous gene for A20 by acting on an endogenous promoter). The agent may include an exogenous promoter. The agent may inhibit an endogenous mechanism ordinarily functioning to limit A20 production. The agent may include a substance that increases the physiological level(s) of A20 in one or more tissues, or systemically, by inhibiting a pathway that leads to the removal, degradation or inactivation of A20. Cognate phrases of "agent that upregulates A20", for example "agents that upregulate A20", or "agent upregulating A20", should be read in the light of this definition.

The term "administering" as used herein is defined as the action of introducing a therapeutic molecule, drug, biologic, gene therapy or other agent into the body of a subject in need of treatment, including but not limited to oral dosing, parenteral dosing including injection, intraperitoneal dosing, transdermal dosing, intranasal dosing, or implantation by surgical or other means.

The term "expression system" as used herein is a genetic composition intended to be introduced into the cells of a subject, including at least one gene and a means of controlling the transcription of the gene in the subject, for example, by the use of a promoter element in the genetic composition utilizing methods and materials well known to those skilled in the art of molecular and synthetic biology.

In some embodiments, administering A20 comprises administering A20 protein to a subject, for example, in a composition (e.g., pharmaceutical composition) comprising the A20 protein.

Gene therapy involves delivering a nucleic acid that encodes the A20 protein to a subject, for example, in a composition (e.g., pharmaceutical composition) comprising the nucleic acid that encodes the A20 protein.

A "subject" is a human, or animal including but not limited to a dog, cat, horse, cow, pig, sheep, goat, chicken, rodent (e.g., rat or mouse), primate (e.g., monkey), and fish. Preferred subjects are human subjects. The human subject may be a pediatric, adult or a geriatric subject.

In some embodiments, A20 is delivered to a subject, for example by intravenous injection, intramuscular injection, intraperitoneal injection, intrathecal injection, adsorption through an epithelial tissue, orally, rectally, intranasally or The A20 is delivered to a specific tissue(s) or organ(s), including but not limited to the liver, . In some embodiments, A20 is delivered in the form of naturally-derived or synthetic A20 (Production Technology of Recombinant Therapeutic Proteins, Chiranjib Chakraborty Biotech Books/Daya Publishing House, New Delhi, India, 2004 ISBN 10: 817622104X / ISBN 13: 9788176221047). In some embodiments, the A20 is delivered after modifying the production of A20 in the cells of target organs or tissues of a patient or subject. (Gene and Cell Therapy: Therapeutic Mechanisms and Strategies, Fourth Edition by Nancy Smyth Templeton (Editor). CRC Press; (January 20, 2015) ISBN-13: 978-1466571990).

In some embodiments the production of A20 in a cell is induced by the introduction of a nucleic acid that encodes the A20 protein. In some embodiments the nucleic acid that encodes the A20 protein is a native or a modified messenger RNA, a single stranded DNA, or a double stranded DNA. In some embodiments, the nucleic acid comprises a promoter. In some embodiments, the nucleic acid comprises an inducible gene. In some embodiments, the nucleic acid comprises an inhibitory sequence. In some embodiments, the nucleic acid serves to inhibit or disable the activity of a constitutive inhibitor of A20 production. In some embodiments, the nucleic acid acts transiently. In some embodiments, the nucleic acid provides long-term therapeutic modification of the level of A20 protein. In some embodiments, the nucleic acid is present within the cell without integration to the subject genome. In some embodiments, the nucleic acid comprises one or more elements that are integrated to the subject genome.

In some embodiments, the production of A20 in a target cell(s) is modified by upregulating the expression of an endogenous gene. In some embodiments, constitutive inhibitors of A20 expression are targeted, such as miRNAs inhibiting A20 expression, and/or targeting A20 anti-sense nucleic acids, thereby disinhibiting production of A20 protein. In some embodiments, the production of A20 in a target cell(s) is modified by upregulating the expression of the endogenous A20 gene by targeting or modifying the endogenous promoter of the A20 gene. In some embodiments, production of A20 in a target cell(s) is modified by modifying the expression of an endogenous gene, which acts to promote or inhibit the expression of the A20 gene and/or the level(s) and/or activity of the A20 protein. In some embodiments, the concentration of the A20 protein is increased by inhibiting pathways that lead to A20 degradation, including but not limited to modifying the post-translational modification of A20 protein by inhibiting its ubiquitination or O-glycosylation. In some embodiments, the expression of the A20 gene or the concentration and/or activity of the A20 protein is modified by a small molecule drug, a biologic drug (including but not limited to an antibody, a hormone, an aptamer or another nucleic acid). In some embodiments, the expression of the A20 gene or the concentration and/or activity of the A20 protein is modified by genetic modification of the cells in the target organ(s) or tissue(s). A non-limiting example includes the use of meganucleases (e.g., homing endonucleaases, LAGLIDADG, I-SceI, I-CreI, transcription activator-like (TAL) effector nucleases (TALENs), megaTALs, zinc-finger nucleases, zinc-finger nickases, and/or the use of clustered regularly interspaced short palindromic repeats (CRISPR) to direct the action of a suitable endonuclease. Non-limiting examples include CRISPR associated protein 9 (Cas9) or Cpf1 from *Francisella novicida.* Targeted Genome Editing Using Site-Specific Nucleases: ZFNs, TALENs, and the CRISPR/Cas9 System 2015th Edition by Takashi Yamamoto (Editor) Springer; 2015 edition (January 6, 2015) ISBN-13: 978-4431552260.

In some embodiments, the expression or production of A20 is increased by 5% 10%, 20%, 30%, 40%, 50%, 60%, 70%, 80%, 90%, 100%, 200%, 300%, 400%, 500% or more.

In some embodiments, therapy is delivered as naked nucleic acid. Non-limiting examples include chemical transfection, lipofection, electroporation, physical stress to cells (including but not limited to microfluidic processing, heat shock and sonication/sonoporation), nucleofection laser microbeam cell surgery, hydrodynamic injection, magnetic assisted transfection and transduction, biolistic particle delivery or nanoparticle or nanoprojectile assisted transfection. Non-Viral Gene Delivery Vectors: Methods and Protocols (Methods in Molecular Biology) 1st ed. 2016 Edition by Gabriele Candiani (Editor), Humana Press; 1st ed. 2016 edition (July 10, 2016), ISBN-13: 978-1493937165.

In some embodiments, therapy may be delivered in the form of modified mRNA or in the form of long non-coding RNAs (IncRNA). In some embodiments, the therapy is delivered by a nucleic acid vector derived from a virus, or comprising a virus-like particle. In some embodiments, the nucleic acid vector comprises a genetic element(s) and/or a protein(s) from adenovirus, lentivirus, herpes virus, retrovirus, *Vaccinia,* or adeno-associated virus. In some embodiments, the nucleic acid vector comprises a genetic element(s) and/or a protein(s) from simian immunodeficiency virus, vesicular stomoatitis virus, HSV-1, HSV-2, Varicella zoster, Epstein-Barr Virus, Cytomegalovirus, a picronavirus with tropism for a target tissue (e.g., Hepatitis A virus), a hapadnavirus with tropism for a target tissue, for example Hepatitis B virus, such as use of a vector comprising HBVpreS-lipopeptides (Hepatitis B virus hepatotropism is mediated by specific receptor recognition in the liver and not restricted to susceptible hosts., Schieck A, Schulze A, Gähler C, Müller T, Haberkorn U, Alexandrov A, Urban S, Mier W. Hepatology. 2013 Jul;58(1):43-53. PMID) and/or a flavivirus with tropism for a target tissue (e.g., Hepatitis C virus).

In some embodiments, the viral vector is constructed using a genetic element(s) and a protein(s) to make a recombinant vector that has minimal toxicity at pharmaceutically effective doses. In some embodiments, the viral vector is constructed using a genetic element(s) and a protein(s) to make a recombinant vector that has minimal immunogenicity at a pharmaceutically effective dose. In some embodiments, the viral vector is constructed using a genetic element(s) and a protein(s) to make a recombinant vector that has minimal immunogenicity at pharmaceutically effective repeated doses. In some embodiments, the viral vector is constructed using a genetic element(s) and a protein(s) to make a recombinant vector that causes minimal infection and/or transduction of cells, tissues, and organs that are not targeted by the specific tropism of the vector. In some embodiments, the vector is constructed with a capsid having functional homology or identity with the capsid of AAV8. Those skilled in the art will appreciate that the AAV8 serotype provides enhanced tropism for liver tissue, and thus preferential transduction of hepatocytes. In some embodiments, the AAV8 capsid is engineered into the AAV2 genome to produce an AAV2/8 recombinant vector for the therapeutic element. A non-limiting example includes an A20 expression cassette. For example, a hemaglutinin A tagged human A20 cDNA is inserted into the multiple cloning site (MCS) of an AAV plasmid under a given promoter. The AAV plasmid comprises two AAV inverted terminal repeat (ITR) sequences that flank the promoter and the MCS (one left ITR and one right ITR). HA-A20 AAV plasmid is then co-transfected with 1) a plasmid that carries the rep and cap genes of AAV (notably the rep gene can be derived from one AAV serotype whereas the cap gene can come from another, therefore enabling the generation of hybrid AAV vectors) , and 2) a helper plasmid that provides the helper genes isolated from adenoviruses into a packaging or producer cell line, typically HEK293. Homologous recombination ensues in dividing cells, leading to the generation of a recombinant AAV encoding the transgene of interest for instance human A20.

In some embodiments, a cell is transfected with a therapeutic nucleic acid by transfection with naked DNA or with a viral vector *in vivo* (e.g., a cell is treated in the subject). In some embodiments, the therapeutic viral vector is introduced by intramuscular injection or intravenous injection (including the hepatic portal vein), intra-arterial injection including the hepatic artery, or intrathecal injection, or subcutaneous injection, or by intraperitoneal injection, or by direct injection into a target tissue or organ. In some embodiments, a cell is harvested from the subject and transfected with naked DNA, or with a viral vector *ex vivo,* prior to reintroduction to the subject (e.g., the subject's own cell(s) is subject is modified *in vitro* prior to autologous cell transfer to the subject). In some embodiments, a cell of heterologous origin is engineered to effect expression of A20 or enhanced expression of A20, or enhanced function of A20, or decreased degradation of A20, and are then subsequently transferred to a subject in need of therapy (e.g., by heterologous cell therapy). In some embodiments, the cell of heterologous origin is human. In some embodiments, the cell of heterologous origin is a human stem cell. In some embodiments, the cell of heterologous origin is a synthetic cell. In some embodiments, the cell is an animal cell genetically edited to be safe and compatible with a human.

In some embodiments, the therapy comprises a recombinant adenovirus (rAd). In some embodiments, the recombinant adenovirus comprises a gene for A20. In some embodiments, the recombinant adenovirus comprises an expression system for enhanced expression of A20 (rAd.A20; an expression system capable of overexpression of A20). In some embodiments, a therapeutically effective dose of the rAd is 1×10⁶ multiplicity of infection (Mol). In some embodiments, a therapeutically effective dose of the rAd is 1×10⁷ multiplicity of infection (Mol) . In some embodiments, a therapeutically effective dose of the rAd is 1×10⁸ MoI, or 1×10⁹ MoI, or 1×10¹⁰ MoI, or 1×10¹¹ MoI, or 1×10¹² MoI.

In some embodiments, the therapy induces euglycemia in a diabetic subject within one week of initiation of therapy. In some embodiments, the therapy induces euglycemia in a diabetic subject or patient within two weeks of initiation of therapy, or within one month of initiation of therapy, or within three months of initiation of therapy.

In some embodiments, fasting blood glucose level(s) in a subject is reduced from greater than 130 mg/dL to between 100 and 130 mg/dL. In some embodiments, the fasting blood glucose level(s) in a subject is reduced from greater than 130 mg/dL to less than 100 mg/dL, or less than 110 mg/dL. In some embodiments, the fasting blood glucose level(s) is reduced by more than 250 mg/dL, or by more than 150 mg/dL, or by more than 50 mg/dL, or by more than 25 mg/dL, or by between 10 and 15 mg/dL. In some embodiments, a reduction in fasting blood glucose levels in a subject of up to 250 mg/dL below pretreatment levels is sustained for 30 days following treatment. In some embodiments, a reduction in fasting blood glucose levels of up to 250 mg/dL below pretreatment levels is sustained for 100 days following treatment. In some embodiments, a reduction in fasting blood glucose levels of up to 250 mg/dL below pretreatment levels is sustained for 300 days following treatment. In some embodiments, a reduction in fasting blood glucose levels of up to 250 mg/dL below pretreatment levels is sustained for more than one year following treatment. In some embodiments, fasting blood glucose levels of a diabetic subject are maintained below 150 mg/dL.. for 30 days following treatment. In some embodiments, fasting blood glucose levels of a diabetic subject are maintained below 150 mg/dL for 100 days following treatment, or for 300 days following treatment, or for more than one year following treatment. In some embodiments, fasting blood glucose levels of a diabetic subject are maintained below 130 mg/dL for 30 days following treatment, or for 100 days following treatment, or for 300 days following treatment, or for more than one year following treatment. In some embodiments, fasting blood glucose levels of a diabetic subject are maintained below 126 mg/dL for 30 days following treatment, or for 100 days following treatment, or for 300 days following treatment, or for more than one year following treatment. In some embodiments, fasting blood glucose levels of a diabetic subject are maintained below 100 mg/dL for 30 days following treatment, or for 100 days following treatment, or for 300 days following treatment, or for more than one year following treatment.

In some embodiments, a subject shows an approximately 200 mg/dL decrease in fasting blood glucose levels for at least 20 weeks or more following therapy.

In some embodiments, the transgene induced expression of A20 as a result of the gene therapy persists for at least 2 weeks following therapy. In some embodiments, the transgene induced expression of A20 as a result of the gene therapy persists for at least 3 weeks, or at least 4 weeks, or at least 5 weeks, or at least 6 weeks, or at least 7 weeks, or at least 8 weeks, or at least 9 weeks, or at least 10 weeks, or at least 11 weeks, or at least 12 weeks, following therapy. In some embodiments, transgene induction of A20 persists for 3 months, 4 months, 5 months, 6 months, 8 months, 12 months, 18 months, 2 years, 3 years, 5 years, 10 years, 20 years, 30 years or more.

In some embodiments, the expression of A20 in the targeted cell(s) is driven by the inclusion of promoter sequences known in the art. In some embodiments, the promoter is specific to the targeted tissue. In some embodiments, the targeted tissue is the liver and the promoter is thyroxin binding globulin promoter. Those skilled in the art will appreciate that this promoter is hepatocyte specific, thus preferentially and efficiently driving transgene expression in the liver.

In some embodiments, the therapy results in a normalized tolerance to acute increase in blood glucose (e.g., in response to a meal or glucose tolerance test) in previously hyperglycemic (diabetic) subject(s). In some embodiments, when the therapy is administered to a diabetic subject who is subsequently given a dose of glucose (orally and/or intravenously), the subject shows peak glucose level comparable to non-diabetic subject receiving the same glucose challenge. In some embodiments, when the therapy is administered to a diabetic subject who is subsequently given a dose of glucose (orally and/or intravenously), the subject shows a glucose level 30 minutes after glucose dosing that is comparable to a non-diabetic subject receiving the same challenge dose of glucose. In some embodiments, when the therapy is administered to a diabetic subject who is subsequently given a dose of glucose (orally and/or intravenously), the subject shows a glucose level 60 minutes after glucose dosing that is comparable to a non-diabetic subject receiving the same challenge dose of glucose. In some embodiments, when the therapy is administered to a diabetic subject who is subsequently given a dose of glucose (orally and/or intravenously), the subject shows a glucoses level 120 minutes after glucose dosing that is comparable to a non-diabetic subject receiving the same challenge dose of glucose. In some embodiments, when the therapy is administered to a diabetic subject who, prior to therapy, had blood glucose level(s) above 180 mg/dL at the 1 hour measurement on administration of the WHO standardized oral glucose tolerance test (OGTT), subject shows a blood glucose level(s) below 180 mg/dL at the 1 hour measurement on the OGTT following therapy. In some embodiments, when the therapy is administered to a diabetic subject who, prior to therapy, had blood glucose level(s) above 140 mg/dL at the 2 hour measurement on administration of the WHO standardized oral glucose tolerance test (OGTT), a subject shows a blood glucose level(s) below 140 mg/dL at the 2 hour measurement on the OGTT following therapy. In some embodiments, when the therapy is administered to a diabetic subject who, prior to therapy, had a blood glucose level(s) above 200 mg/dL at the 2 hour measurement on administration of the WHO standardized oral glucose tolerance test (OGTT), a subject shows a blood glucose level(s) below 140 mg/dL at the 2 hour measurement on the OGTT following therapy. In some embodiments, when the therapy is administered to a diabetic subject who, without therapy, had a blood glucose level(s) above 300 mg/dL, after challenge with glucose showed a blood glucose level(s) 2 hours after glucose challenge that were at least 50 mg/dL lower, or at least 100 mg/dL lower, or at least 150 mg/dL lower, or at least 200 mg/dL lower than the level(s) seen 2 hours post glucose challenge prior to treatment with the therapy of the present invention.

In some embodiments, glucose challenge in a previously diabetic subject subsequently treated with the therapy as presently disclosed prompted little or no increase in insulin upon challenge with glucose. In some embodiments, glucose challenge in a non-diabetic subject subsequently treated with the therapy as presently disclosed showed decreased insulin levels upon challenge with glucose compared to non-diabetic subjects not treated with the therapies disclosed herein. In some embodiments, the reduction in glycemia following therapy did not lead to hypoglycemia either during fasting or following feeding. One skilled in the art will thus appreciate that A20 therapy provides both improved and self-limiting glycemic control by ameliorating hyperglycemia without causing hypoglycemia.

In some embodiments, the therapy(ies) herein disclosed leads to decreased hepatic expression of genes associated with gluconeogenesis. In some embodiments, the disclosed therapy(ies) results in lowering the levels of expression of glucose-6-phosphatase in the subject. In some embodiments, the disclosed t therapy(ies) results in lowering the levels of hepatic expression of phosphoenolpyruvate carboxykinase in the subject. In some embodiments, the disclosed therapy(ies) results in lowering the levels of hepatic expression of glucose-6-phosphatase in the subject. In some embodiments, the disclosed therapy(ies) results in lowering the levels of hepatic expression of nuclear receptor peroxisome proliferator-activated receptor gamma coactivator 1-alpha in the subject. One skilled in the art will appreciate that the A20 therapy(ies) presently disclosed can thus downregulate transcription of gluconeogenic pathways in the liver.

In some embodiments, the therapy(ies) disclosed leads to an increase in the hepatic storage of glycogen. For example, significantly higher glycogen levels were detected in the liver of diabetic mice two to three days after the restoration of glycemic control as a result of treatment with the therapy disclosed in the present application compared to diabetic mice treated with a control not leading to higher levels of A20 protein. Those skilled in the art will appreciate that such an embodiment represents a novel method of controlling glycogen storage. Those skilled in the art will further appreciate that this mechanism accounts for the observation that a subject treated with the therapy(ies) presently disclosed do not suffer hypoglycemia as the improved level of glycogen storage potentiates the ability to respond to falling blood glucose by activating glycogenolytic pathways in the liver to utilize such glycogen stores to balance demand for blood glucose.

In some embodiments, the therapy(ies) disclosed herein results in modification of the expression and activity of glycogen synthase in the liver and/or in skeletal muscle. In some embodiments, the disclosed therapy(ies) leads to increased expression of glucose transporters (GLUT) in the liver. For example, hepatic levels of both GLUT1 and GLUT2 are increased in response to the A20 boosting therapy of the present invention. Those skilled in art will appreciate that GLUT2 is the primary insulin-independent driver of glucose uptake by hepatocytes, which provides a functional mechanism to explain some of the mechanism of action of the instantly disclosed therapy(ies), in that hepatocytes function as a sink for excess blood glucose while local elevation of glucose concentration in hepatocytes helps to push the pathway towards glycogen synthesis.

In some embodiments, therapy(ies) disclosed herein leads to increased expression of glucose transporters (GLUT) in skeletal muscle, comprising an increase in the level of glucose transporter GLUT4 (the primary glucose transport in muscle) in skeletal muscle, and/or an in peroxisome proliferator-activated receptor gamma coactivator 1-alpha (PGC-1α), known to regulate the expression of GLUT4, and/or the expression of GLUT1, in skeletal muscle (an accessory glucose transporter in muscle tissue). In some embodiments, the systematic upregulation of PGC1-1α can be used to address other disease states impacted by PGC-1α, including disease states potentially linked to mitochondrial biogenesis, including Parkinson's Disease, Amyotrophic Lateral Sclerosis, Huntingdon's Disease, or other neurodegenerative diseases impacted by mitochondrial dysfunction. (Zheng et al., Global PD Gene Expression (GPEX) Consortium, PGC-1α, a potential therapeutic target for early intervention in Parkinson's disease. Sci Transl Med. 2010 Oct 6;2(52); Procaccio et al., Perspectives of drug-based neuroprotection targeting mitochondria. Rev Neurol (Paris). 2014 May;170(5):390-400; Eschbach et al., PGC-1α is a male-specific disease modifier of human and experimental amyotrophic lateral sclerosis. Hum Mol Genet. 2013 Sep 1;22(17):3477-84; Török et al., mRNA expression levels of PGC-1α in a transgenic and a toxin model of Huntington's disease. Cell Mol Neurobiol. 2015 Mar;35(2):293-301). For example, while previously the function of A20 in the liver was understood to extend to local anti-inflammatory, anti-apoptotic, and pro-regenerative functions, the present disclosure unexpectedly reveals that an intervention increasing the effect of A20 in the liver not only positively influences local hepatic glucose metabolism, but also systematically impacts the regulation of gene expression in other organs and tissues (see, for example Fig. 3) including PGC-1α. Those skilled in the art will recognize that PGC-1α is a transcription coactivator known to be a key regulator of energy metabolism within the cell. Because PGC-1α is known to be involved in the regulation of a broad range of factors related to metabolic diseases, including diabetes, obesity, lipid metabolism, metabolic syndrome and cardiomyopathy, it has been previously considered as a direct target for pharmaceutical approaches to diabetes and other metabolic disorders such as obesity. Because PGC-1α stimulates mitochondrial biogenesis, the key engine of cellular metabolism, the unexpected impact of hepatic expression of A20 on the transcription of PGC-1α in non-hepatic tissues is likely to have much broader implications for the regulation of metabolism and potential treatment of disorders of metabolism than the remarkable restoration of glucose homeostasis demonstrated here. In addition to the restoration of glucose homeostasis, in some embodiments, the A20 therapy disclosed herein is useful in the treatment of other dysfunctions of metabolic homeostasis, wherein metabolic homeostasis is taken as the desirable and healthy regulation of metabolism and metabolites, and its dysfunction is understood to be an undesirable and/or unhealthy imbalance of metabolism and/or metabolites. Non-limiting examples include the distribution, storage and utilization of glucose, glycogen, fatty acids, triglycerides, lipids, lipoprotein, iron and calcium. In some embodiments of the methods disclosed herein, interventions based on modifying the expression of A20 can be used to treat a broader set of potentially interrelated pathologies due to their common relationship to disorders of fundamental metabolic processes at the tissue, cellular, and even mitochondrial level. Because those skilled in the art will also appreciate that PGC-1α has been previously linked to certain treatment-refractory degenerative diseases (including, but not limited to Huntington's disease, Parkinson's Disease, and/or Amyotrophic Lateral Sclerosis) which are also known or suspected to involve mitochondrial dysfunction, one skilled in the art would understand that the methods disclosed herein can be used in remediating, treating or curing such diseases.

In some embodiments, a subject treated by the disclosed method(s) shows increased expression of lipocalin-13 (LCN13), which those skilled in the art will recognize as an anti-diabetic, glucose-regulating agent.( Cho KW, Zhou Y, Sheng L, Rui L. Lipocalin-13 regulates glucose metabolism by both insulin-dependent and insulin-independent mechanisms. Mol Cell Biol. 2011 Feb;31(3):450-7) In some embodiments, using the method(s) taught by the present disclosure, a subject shows decreased expression of retinol binding protein 4 (RBP4). Those skilled in the art will recognize RBP4 is a pro-diabetic agent associated with a variety of disease states linked to metabolic dysregulation. (Hu H, Xu M, Qi R, Wang Y, Wang C, Liu J, Luo L, Xia L, Fang Z. Sitagliptin downregulates retinol-binding protein 4 and upregulates glucose transporter type 4 expression in a Type 2 diabetes mellitus rat model. Int J Clin Exp Med. 2015 Oct 15;8(10):17902-11) In some embodiments, the method(s) presently taught are associated with both increase in hepatic expression of LCN13 and decreased expression of RBP4, which those skilled in the art will recognize is a unique combination of effects not previously linked to modulation of glycemic control in Type 1 diabetes, and not previously associated with any therapeutic benefit in terms of improved glycemic control in the context of Type 1 diabetes.

In some embodiments, the methods taught by the present disclosure can be used to treat subjects or patients suffering from autoimmune diabetes (that is Type 1 diabetes) wherein treatment outcomes include, but are not limited to, restoration of euglycemia, establishment of fasting blood glucose level below 100 mg/dL, or establishment of fasting blood glucose level below 126 mg/dL, or the reduction of fasting blood glucose level(s) by between 25 and 50 mg/dL, or between 50 and 100 mg/dL, or between 100 and 200 mg/dL, or between 200 and 500 mg/dL, and/or slower increase in blood glucose level in response to glucose tolerance testing, and/or lower peak blood glucose level in response to glucose tolerance testing, and/or more rapid return towards baseline blood glucose in response to glucose tolerance testing.

In some embodiments, the disclosed method(s) improves the fasting blood glucose of Type 2 diabetic (T2D) subject (e.g., those with a metabolic disorder comprising decreased insulin production due to insulin resistance and/or hyperglycemia). In some embodiments, the disclosed method(s) results in the fasting blood glucose of T2D subject to be at or below 110 mg/dL. In some embodiments, the disclosed method(s) causes and/or result(s) in the blood glucose of T2D subject at two hours after glucose challenge in OGTT to be lower than prior to treatment. In some embodiments, the disclosed method(s) causes and/or results in the blood glucose of T2D subject at two hours after glucose challenge in OGTT to be lower than 140 mg/dL. In some embodiments, the disclosed method(s) causes the glycated hemoglobin level of T2D subject to decrease with respect to levels prior to treatment or absent treatment. In some embodiments, the present method(s) causes and/or results in the glycated hemoglobin level of T2D subject to decrease below 48 mmol/mol. In some embodiments, the disclosed method(s) causes and/or results in the glycated hemoglobin level of T2D subject to decrease below 42 mmol/mol.

In some embodiments, the disclosed method(s) can improve, ameliorate one or more sign or symptom of, or cure Type1 diabetes, Type 2 diabetes, gestational diabetes, maturity onset diabetes of the young, Rabson-Meendenhall Syndrome, Donahue Syndrome, diabetic pathophysiology as a result of mitochondrial DNA mutations, diabetes caused by genetic defects in insulin processing or insulin action, such as defective proinsulin conversion, mutations to the insulin gene itself or in the insulin receptor, exocrine defects of the pancreas causing diabetes, diabetes secondary to chronic pancreatitis, pancreatectomy, pancreatic neoplasia, diabetes related to cystic fibrosis, hemochromatosis or fibrocalculous pancreatopathy; endocrinopathies leading to diabetes, acromegaly associated diabetes, diabetes associated with Cushing's syndrome, Down's syndrome, Klinefelter syndrome, and Turner syndrome, hyperthyroidism, pheochromocytoma, glucagonoma, infection with coxsackievirus B or CMV or HCV, lipodystrophy, diabetes resulting from side effects and/or toxicity of drugs including statins, thyroid hormone, glucocorticoids or beta-andregenic agonists, pentamidine, nicotinic acid, didanosine stavudine, zidovudine, indinavir, lopinavir/ritonavir, or diabetes secondary to exposure to toxins such as dioxin.

The method disclosed herein represent a major improvement in the therapy for all forms of diabetes, in that that they avoid the risk of hypoglycemia as well as avoid the risk and negative impact to lifestyle of traditional diabetes therapies requiring intervention daily or more frequently, particularly insulin therapy. One skilled in the art will appreciate that the present invention has the potential to replace insulin therapy as well as other diabetes therapies, or minimize the need for such therapies with as little as a single dose of A20 promoting therapy.

### EXAMPLES

### Recombinant adenoviral (rAd) mediated gene transfer of A20 in livers of STZ-diabetic C57BL/6 mice restores fasting euglycemia and normalizes the glucose tolerance test, in an insulin-independent manner and without causing hypoglycemia.

Six-week old male C57BL/6 mice received 5 consecutive (once a day) intraperitoneal (IP) injections of 60mg/kg of the β-cell toxic agent streptozotocin (STZ) diluted in citrate buffer¹. Control mice were treated with citrate buffer (CIT). Blood glucose levels were measured on a weekly basis after rAd administration with a glucometer and following 12h overnight (O/N) fasting. Diabetes was confirmed by 3 consecutive fasting glycemias ≥250 mg/dL. Five to six weeks after diabetes was established, STZ and CIT-treated mice were injected intravenously with 1×10⁹ multiplicity of infection (MOI) of rAd.A20 or control rAd.βgal (beta-galactosidase). Remarkably, 83% of rAd.A20 STZ (n=10 of 12), but none of rAd.βgal STZ (n=8) mice became euglycemic within one week of rAd injection. Fasting blood glucose levels were at 100-130 mg/dL in A20 and >400mg/dL in βgal-treated mice (FIG. 1A). This appears to be the first demonstration that overexpression of A20 in livers of STZ-treated diabetic mice rapidly restores euglycemia in fasting conditions. Fasting blood glucose levels consistently remained <150 mg/dL for 100 days after rAd.A20 injection in 2 out of 3 STZ-treated mice followed long-term (FIG. 1B). This indicates that A20's anti-diabetic effect likely persists beyond its hepatic expression, i.e. rAd-induced transgene expression in the liver usually lasts 4-6 weeks after transduction².

Next, a glucose tolerance test (GTT) was performed in STZ mice treated with rAd.A20 to check whether euglycemia was only achieved in fasting conditions or if it would sustain a glucose load. A20 and βgal-treated STZ mice were injected IP, after O/N fast, with a 2g/kg solution of 20% glucose. Blood glucose, as well as serum insulin levels (Ultrasensitive Mouse Insulin ELISA Mercodia AB Sweden) were measured 30, 60 and 120 min later. The data indicate that diabetic mice that became euglycemic a week after administration of rAd.A20 had a normalized GTT curve, i.e. similar to that of rAd.βgal CIT controls (FIG. 1C). Glycemia in rAd.A20 STZ mice averaged 111 mg±8 /dL at baseline, peaked to 210±90 mg/dL 30 min after glucose load and decreased to 167±34 mg/dL 120 min post-load. This contrasted with a totally abnormal GTT curve in rAd.βgal STZ mice whose glycemia was consistently >300mg/dl (FIG. 1C).

Remarkably, normalization of the GTT curve in rAd.A20 STZ mice was not coupled with increased serum insulin levels. Insulin serum levels in rAd.A20 STZ mice were, as in hyperglycemic rAd.βgal STZ mice, at the detection limit of the assay (FIG. ID). CIT-treated mice increased their serum insulin levels in response to the glucose load. However, this increase was much lower in rAd.A20 CIT vs. rAd.βgal CIT mice, and corresponded with a flatter GTT curve in these mice. This result suggests that even non-diabetic A20-treated mice benefit from an insulin-sparing effect. By immunohistochemistry (IHC), it was confirmed that there was no defined islets in the pancreas of rAd.A20 STZ and rAd.βgal STZ-treated mice. Their pancreas only showed some scant insulin staining (FIG. 2).

Notably, none of the A20-treated mice experienced hypoglycemia in either fed state or after overnight (O/N) fast. This result indicates that the A20's positive effect on glycemic control is self-limiting.

### Recovery of glucose homeostasis in A20-treated diabetic mice corresponds with decreased liver expression of gluconeogenic genes, increased hepatic glycogen storage, and increased expression of glucose transporters (GLUT) in liver and skeletal muscles.

In normal conditions, glucose metabolism is regulated by several key metabolic pathways including: *de novo* glucose production (gluconeogenesis), 80% of which occurs in the liver; breakdown of glycogen storage (glycogenolysis), mostly from hepatic stores, and peripheral glucose uptake and storage by skeletal muscles and adipose tissue. Classically, hepatic gluconeogenesis and glycogenolysis are under the concerted control of insulin and glucagon that activate a number of key and often rate-limiting enzymes that regulate these processes³. The present results indicate that liver-expressed A20 regulates expression of genes involved in hepatic glucose production and metabolism in a way that improves glucose homeostasis, but in an insulin-independent manner. A transcriptional profiling of rAd.A20 STZ and rAd.βgal STZ livers, 10 days after rAd. injection and 2-3 days after normalization of fasting glycemia in A20-treated mice showed significantly lower mRNA levels of the key gluconeogenic genes, glucose-6-phosphatase (G6Pase) and phosphoenolpyruvate carboxykinase (PEPCK) in livers of rAd.A20 STZ vs.rAd.βgal STZ mice (FIG. 3A). Transcription of gluconeogenic genes in the liver is, at least in part, controlled by expression/activity of the nuclear receptor peroxisome proliferator-activated receptor gamma coactivator 1-alpha (PGC1α)⁴. Hepatic mRNA levels of PGC1α increase in response to fasting to promote transcription of gluconeogenic genes⁵⁻⁷. Indeed, transcript levels of PGC1α were shown to be significantly lower in rAd.A20 STZ vs. rAd.βgal STZ mice (p= 0.0261, FIG. 3A).

Despite reduced hepatic gluconeogenesis, none of the STZ-treated mice that became euglycemic upon rAd.A20 injection, experienced hypoglycemia. This observation suggests that the system was able to self-regulate. Storage of glucose under its glycogen form in the liver is key to the body's ability to rapidly respond to hypoglycemia by initiating glycogenolysis to release and replenish circulating glucose. Using a commercially available glycogen Periodic Acid Schiff (PAS) staining kit, significantly higher glycogen levels in rAd.A20 STZ vs. rAd.βgal STZ livers, 2-3 days after these mice had normalized their blood glucose levels were observed (FIG. 3B). This is a newly discovered function of A20 that likely contributes to the "no" or very low risk of hypoglycemia that associates with A20-mediated cure of diabetes. Glycogen synthesis is usually controlled by opposing effects of glucagon and insulin and requires the coordinated activation of a number of enzymes⁸. Glycogen synthase (GS) is the key enzyme required for glycogen synthesis. Whether liver-expressed A20 impacts expression and activity of this enzyme in liver and skeletal muscle, even when insulin is absent will be further investigated.

A20 also significantly increased hepatic mRNA levels of the glucose transporters GLUT2 and GLUT1 (FIG. 3C). GLUT2 is the primary and most abundant GLUT for non-insulin entry into hepatocytes³. This could result in more glucose entry in A20 vs. βgal-treated hepatocytes.

However, neither decreased gluconeogenesis nor enhanced hepatic glycogen storage (only 33% of ingested glucose shuttles through the liver) can account for normalization of the GTT. Rather, normalized GTT is bound to reflect improved peripheral glucose uptake, such as by skeletal muscles and adipose tissue. Significantly higher mRNA levels of the dominant glucose transporter GLUT4 and its transcriptional regulator, PGC1α, as well as of GLUT1 in skeletal muscles of A20 vs. βgal-treated mice were observed (FIG. 4A). This supports a hypothesis of increased glucose uptake by skeletal muscles. The function of GLUT4 is mostly regulated by its insulin-induced translocation and fusion to the cellular membrane¹¹⁻¹⁴. Increased GLUT4 mRNA levels need to translate into both higher protein levels and higher membrane localization in order for glucose uptake to be enhanced. The results showing higher glycogen stores in muscles of rAd.A20 vs. rAd.βgal-treated mice support this (FIG. 4B).

### A20 overexpression in livers of STZ-treated diabetic mice increases hepatic levels of anti-diabetic 13 (LCN13) and decreases levels of pro-diabetic retinol binding protein 4 (RBP4).

Because liver-expressed A20 caused remote changes in skeletal muscle mRNA levels, it was thought that A20 was influencing expression and/or release of hepatocyte-produced regulator(s) of glucose uptake. In revisiting transcriptome data from rAd.A20 vs. rAd.βgal transduced livers gathered from nondiabetic mice to gauge A20's impact on the liver regenerative process, two plausible candidates were uncovered, lipocalin-13 (LCN13) and retinol-binding protein4 (RBP4), whose expression was differentially modulated by overexpression of A20.

Livers from CIT and STZ-treated mice were analyzed and hepatic mRNA and protein levels of anti-diabetic LCN13 were found to be significantly higher in CIT and STZ A20 vs. βgal-treated mice (FIGs. 5A, 5C). Conversely, mRNA levels of pro-diabetic RBP4 were lower in both CIT and STZ A20 vs. βgal-treated mice (FIG. 5B). This decrease was particularly significant in the STZ-A20 group. LCN are secreted solute carrier proteins that transport hydrophobic molecules such as FA and other phospholipids, retinol, and pheromones^{15,16}. Although mostly known for their shuttling of pheromones and their influence on interactive behavior, a number of LCN, including LCN-13, and RBP4 have been recently recognized to respectively yield a positive or negative impact on lipid and glucose metabolism, and insulin sensitivity. However, these effects were described in the context of obesity, insulin resistance, and type 2 diabetes ¹⁷⁻²⁰. Neither LCN13 nor RBP4 have so far been implicated in influencing glycemic control in experimental models of T1D, as demonstrated in the STZ/C57BL6 mouse model. A20 appears to be unique in its ability to simultaneously influence both molecules and significantly tip the balance in favor of anti-diabetic LCN13, while depressing that of pro-diabetic RBP4.

### A20 overexpression in the liver of STZ-treated diabetic mice increases circulating levels of anti-diabetic LCN13.

Since liver-expressed A20 caused remote changes in skeletal muscle mRNA levels, it was surmised that it was influencing expression and/or release of a secreted liver-produced regulator(s) of glucose uptake, and subsequently LCN13 was identified as the most plausible candidate. It was previously disclosed herein that hepatic mRNA and protein levels of LCN13 were significantly higher in rAd.A20 vs. control rAd.βgal-treated mice treated mice, whether non-diabetic (citrate, CIT) or diabetic (streptozotocin, STZ). It was then sought to determine whether increased hepatic levels of LCN13 translated into increased circulating serum levels of this classically secreted solute carrier. Using a commercially available LCN13 ELISA, serum levels of LCN13 in CIT and STZ rAd.A20-treated mice and rAd.βgal treated controls were measured. The data outlined herein demonstrated that significantly higher intrahepatic levels of LCN13 in CIT and STZ rAd.A20 mice were associated with significantly higher circulating levels of LCN13, i.e. >20 fold higher in rAd.A20 vs. rAd.βgal treated mice (FIG. 6).

### Recombinant adenoviral mediated gene transfer of A20 in livers of diabetic NOD mice restores euglycemia and improves the GTT curve.

Because auto-immune diabetes is much more difficult to control, we examined whether intravenous administration of rAd.A20 can also improve diabetes in diabetic female NOD mice. In brief, female NOD that developed overt diabetes between 12 and 14 weeks of age, were randomized to receive 2 intravenous injections of either 10⁹ MOI rAd.A20 or rAd.βgal, administered one week apart. Two, rather than one, doses of rAd. were injected in an attempt to respond to the extreme hyperglycemia (>500mg/dL) that these mice experience, which could interfere with A20 expression levels or function. Remarkably, A20-, but not βgal-, treated NOD mice became euglycemic, and showed an improved and rather flattened GTT glycemia curve (FIG. 7). This is the first demonstration that hepatic overexpression of A20 cures autoimmune diabetes. However, the mechanisms underscoring the anti-diabetic effect of A20 also predict that it will achieve similar benefits in T2D.

### Lipidomics screening of rAd.A20 vs. rAd.βgal treated CIT and STZ mice showed significant quantitative and qualitative modulation of the liver lipid profile by A20, and identified several candidate ligands for LCN13.

LCN13 is a secreted solute carrier protein that transport hydrophobic molecules presumed to be fatty acids (FA) or phospholipids. Therefore, the anti-diabetic effect of LCN13 may hence, at least in part, depend on the cargo it binds.

In one embodiment of the proposed therapy, A20 could also modulate the putative FA or phospholipid cargo(s) of LCN13 to influence its anti-diabetic function. To address this question, the influence of A20 on the lipid composition of CIT and STZ mice was explored. Briefly, a Thermo Fisher Q-exactive instrument was used to probe, by liquid chromatography/mass spectrometry (LC-MS) in negative and positive ionization mode, for changes in the liver lipidome of rAd.A20 vs. rAd. βgal-transduced livers of CIT and STZ mice. The data was analyzed using the LipidSearch^{™} software for identification and relative quantification of lipids.

1204 lipid species representing all lipid classes were identified (FIG. 8). Differences in lipid composition between rAd.A20 and rAd.βgal were qualitatively and quantitatively much more pronounced in diabetic than nondiabetic mice.

First, there were 40 lipid species in the CIT group and 88 in the STZ group that were significantly lower (<2-fold, p<0.05) in rAd.A20 vs. rAd.βgal livers; 17 of which were common to both CIT and STZ groups (FIG. 9).

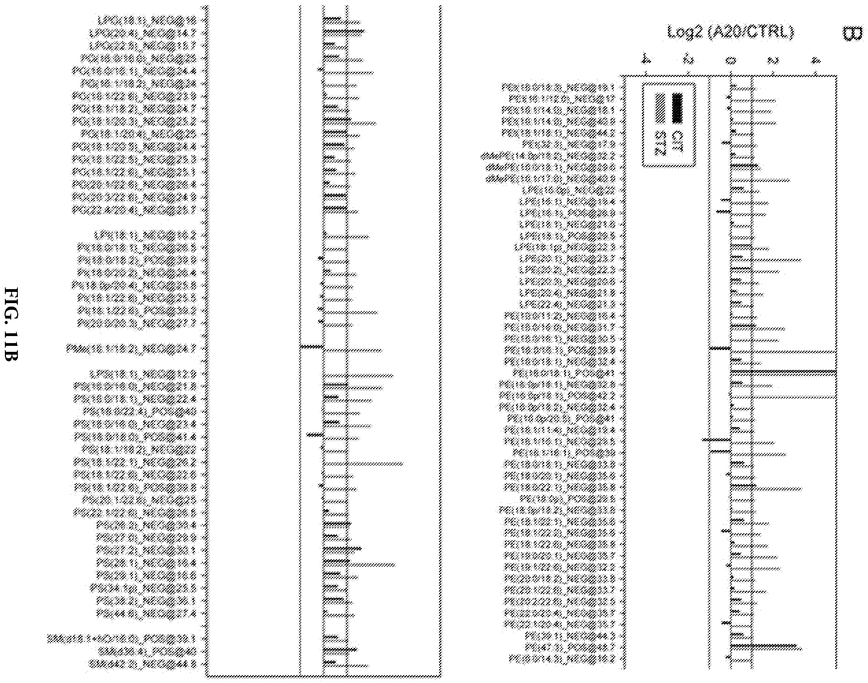

Second, 7 lipid species in the CIT group and 182 in the STZ group were recorded that were significantly increased (>2-fold, p,0.05) in rAd.A20 vs. rAd.βgal treated livers. Only 5 of these lipids were common to CIT and STZ (FIG. 9). These 5 lipid species were the most promising LCN13 binding antidiabetic candidates since the effect of A20 on insulin sparing and improving the glucose tolerance test (GTT) was evident in both CIT and STZ mice. Notably, these 5 lipids were almost undetectable in rAd.βgal CIT and STZ livers, while significantly enriched by in both rAd.A20 CIT and STZ (FIG. 10). The specific enrichment of these lipids in rAd.A20 livers parallels that of LCN13 (10-20 fold). These lipids were identified and confirmed by MS/MS METLIN match as cardiolipin (CL) 22:6/18:1/20:0/22:6; CL85:5; triglyceride (TG) 18:0/18:1/18:1; lisophosphatidylglycerol (LPG) 20:3; and phosphatidylglycerol (PG) 18:3/18:2. However, other lipids whose levels were specifically modulated by A20 in diabetic STZ mice may also represent preferential anti-diabetic LCN13 ligands and cannot be ruled out. These lipids are depicted in FIGs. 11A and 11B.

Third, lipid species that were not identified by LipidSearch^{™}, yet specifically enriched in rAd.A20 livers were also discovered. The most abundant of these lipids with a corresponding mass to charge (m/z) ratio of 580.325 was 2-3 fold higher in rAd.A20 vs. rAd.βgal treated livers (p<0.001) (FIG. 12). This lipid likely corresponds to an oxidized phosphatidylethalonamine (PE) C18:2/C4.

### Prophetic Example

A20 is an obligatory intracellular protein. Hence its use as a therapy in the clinic entails devising safe and efficient gene delivery tools that results in adequate transgene expression in hepatocytes. The discovery of naturally occurring AAV in multiple mammalian species, together with the recent development of novel and efficient hybrid (comprising capsids from different serotypes), chimeric and synthetic recombinant (rAAV) with preferential tissue tropism has revived the promise of gene therapy^{21,22}. The proven safety record, minimal toxicity, and low immunogenicity of these rAAV has propelled their use to the forefront of gene therapy vectors²³.

Notably, the AAV8 serotype capsid shows higher liver tropism than other capsids and enables faster hepatic transgene expression²⁴. Hybrid rAAV comprising the AAV8 capsid combined with the AAV2 genome (rAAV2/8) achieve high transgene expression in livers of rodents, dogs, and non-human primates following iv or portal vein injection^{22,25,26}. Transgene expression lasted for months (even years) after transduction, which is optimal for treating T1D. Indeed, one injection of AAV2/8 could control diabetes for months, possibly years. AAV2/8 based gene therapy vectors are currently used in numerous clinical trials to treat Hemophilia B and hence their implementation in diabetic patients should not pose any regulatory problems ²⁷⁻³⁰. We generated a rAAV2/8 vector expressing an HA-tagged A20 under the control of the hepatocyte-specific Thyroxin Binding Globulin (TBG) promoter. This vector preferentially and rapidly drives hepatocyte-specific transgene expression^{22,32}. Intravenous injection of rAAV2/8.TBG.HA-A20 (1011 viral genome (vg)/mouse) or control rAAV2/8.TBG.GFP yielded high transgene expression in mouse hepatocytes within 1-2 days after the injection (FIG. 13A), and in hepatocytes of miniature swine (10¹² vg/kg) (FIG. 13B), without causing toxicity, as evidenced by the absence of any alteration of the liver functions tests. This A20 vector could be readily used in humans, in particular for the treatment of T1D, and possibly T2D.

Despite all positive indicators from the current use of AAV, repetitive re-dosing may still present some challenges. Hence, whether newly developed gene therapy tools based on modified mRNA or long noncoding RNA (lncRNA) may as efficiently increase A20 expression in hepatocytes is being explored as valuable alternatives to AAV³³⁻³⁵. A fundamental evolution in our approach to translate A20 to the clinic is recognition of the constantly evolving molecular strategies that could be implemented to express A20 in liver cells.

At this stage, there is convincing evidence that overexpression of A20 in hepatocytes restores glycemic control and normalizes GTT, in an insulin-independent manner, and without causing hypoglycemia, in two mouse models of T1D, chemically-induced and auto-immune. This benefit relies on A20 decreasing hepatic gluconeogenesis and increasing glucose uptake and glycogen storage in the liver and muscles. Additionally, a clinically safe vector based on AAV2/8 has been developed to express A20 in the liver, and its use has been validated in pigs. This vector, when manufactured clinic-grade, could readily be used in patients.

The data sets the stage for studies in large animal models of T1D in prelude to clinical translation. Non-human primates (NHP), mostly Cynomolgus monkeys and Rhesus macaques, rendered diabetic by either pancreatectomy or STZ destruction of β-cells, best recapitulate human T1D and its response to therapies³⁶⁻³⁹. From both an ethical and financial perspective, positive outcomes in mice are needed to justify NHP experimentation. Low AAV immunogenicity, added to A20's established ability to decrease immune responses, predicts that NHP treated with AAV-A20 will not develop a neutralizing anti-AAV immune response, and therefore could be successfully re-dosed if needed. As the current regulatory situation is favorable for the clinical use of AAV-based therapies, clinical translation of an A20 therapy to treat T1D will rapidly follow conclusive results in NHP.

### Current Diabetes Therapies

Current therapies that provide stringent control of blood glucose levels have so far been met with limited success for varied reasons, as detailed below. Based on the proposed mechanisms of action of A20, as well as safety, feasibility, and flexibility of the delivery system disclosed herein, AAV-based and liver-directed expression of A20 overcomes many limitations of the other available invasive and non-invasive approaches.

Classic intensive insulin therapy is difficult to implement without imposing drastic life-style changes that lead to non-compliance. Additionally, and perhaps even more troublesome, is the fact that such a strict regimen carries a significant risk for severe hypoglycemia. This is illustrated in a recent study where most deaths in T1D patients who are less than 50 years old were mostly due to (mis)management of diabetes, not cardiovascular complications⁴⁰. None of the mice that became euglycemic after treatment with rAd.A20 disclosed herein experienced hypoglycemia. This is an enormous and surprising advantage over intensive insulin therapy.

Fully automated closed loop systems equipped with sensors for continuous glucose monitoring (CGM), together with pumps for real-time immediate adapted release of insulin and/or glucagon in bi-hormonal devices were developed to enable a sustained control of glycemia levels, with the hope of avoiding glycemic excursions in brittle diabetics⁴¹⁻⁴³. However, despite significant investments in many companies to design and construct these devices, the systems are challenged by many technical hurdles, including: frequent recalibration, periodic change of sensors, and software standardization to adapt to variability in blood glucose levels associated with food intake, exercise, or other interfering health issues. For example, the Medtronics trial of a hybrid closed loop insulin system with a 3CGM reading every 5 min that adjusts insulin levels to achieve a target glycemia 120 mg/dL still requires boluses for meal and also informing the system of exercise. Finally, these systems could be vulnerable to technical glitches and software malfunctions, which is especially problematic in children whose device is remotely controlled by an adult, are costly, and still need FDA approval^{44,45}. Obviously, a simple intravenous injection of AAV.A20 that could achieve similar HblAc target for months and even years, using a vector that has received FDA approval for hemophilia B, and is less costly, has clear advantages.

Islet transplantation that re-emerged in 2000, after being stalled for a couple of decades, after the success of the Edmonton protocol, as a cure for T1D, is scarcely used and for limited indications such as hypoglycemia unawareness. In addition to being an invasive procedure implicating general anesthesia and intra-portal injection of the islets, broader implementation of islet transplantation is mainly limited by the serious infectious and oncogenic side effects of chronic immunosuppression, and scarce supply. Two or three donors are often needed in order to deliver a sufficient islet mass that would restore euglycemia^{46,47}. Safety and low immunogenicity of AAV together with the added hepatoprotective benefit of A20 well out-rank islet cell transplantation.

Despite the progress that has been made over the last decade in the search for a stem cell-based therapy to reprogram multi-potent cells into insulin-producing cells and cure diabetes, this approach still faces significant technical and ethical hurdles that need to be addressed, before it can fulfill its clinical promise⁴⁸. Notwithstanding that, it will likely not resolve the additional confounding issue of insulin resistance, which A20 will most likely circumvent.

Other non-insulin dependent strategies that were recently proposed to achieve target HbAlc levels with lower insulin doses include small molecule inhibitors of sodium glucose co-transporters 1 and 2 (SGLT2, SGLT1) that improve glycemic control by blocking glucose reabsorption in the kidney⁴⁹, and glucagon-like peptide 1 (GLP-1) receptor agonists^{50,51}. Given their mechanism of action, SGLT2 inhibitors as well as GLP1 receptor agonists circumvent insulin resistance and do not seem to provoke hypoglycemia. A number of SGLT2 inhibitors and GLP1R agonists are FDA-approved for the treatment of T2D^{51,52}. Although these drugs improve glycemic control, and reduce vascular and renal complications of diabetes⁵²⁻⁵⁴, their widespread use remains plagued by numerous side effects and limited by the fact that they are still considered adjunct therapies. Side effects of SGLT2 inhibitors include the high occurrence of urinary tract and genital fungal infections⁵⁵, which could be extremely problematic in T1D patients who are already at increased risk for these diseases. Most importantly, SGLT2 inhibitors also increase the incidence of ketoacidosis, a very serious complication that occurs in the absence of hyperglycemia⁵⁶⁻⁵⁹. Based on the latter, the FDA recently issued a warning about this serious side effect of SGLT2 inhibitors, and has halted their use in T1D patients who may be at greater risk than T2D patients for developing ketoacidosis. GLP1R agonists offer the attractive possibility of a weekly dosing regimen, but are not as effective as SGLT2 inhibitors. They also carry a risk for gastro-intestinal side effects including nausea, vomiting, diarrhea, in addition to reports of hypoglycemic episodes when used in combination with the insulin secretagogue sulfonylurea⁶⁰. Furthermore, both SGLT2/SGLT1 inhibitors and GLP-1 receptor agonists remain adjuvant therapies and hence need to be associated with classic anti-diabetic therapies. The data indicate that A20-based therapy may be successful as a monotherapy.

A20-based therapies represent an innovative strategy to improve blood glucose control in T1D and T2D. A20 therapy offers several advantages over all available therapies, i.e., it is highly effective, does not cause hypoglycemia, and is insulin-independent, circumventing insulin resistance. Importantly, it is also easy to implement in the clinic thanks to the established safety profile of novel FDA-approved AAV vectors, with relatively low manufacturing cost. Its simple route and regimen of administration permits a flexible lifestyle, with a single intravenous dose covering numerous months. Based on mouse data, an A20 therapy is likely to be effective as a monotherapy, but if not, will drastically reduce the need for insulin.

### References

1. Zhou Y, Jiang L, Rui L. Identification of MUP1 as a regulator for glucose and lipid metabolism in mice. J Biol Chem. Apr 24 2009;284(17):11152-11159.
2. Arvelo MB, Cooper JT, Longo C, et al. A20 protects mice from Dgalactosamine/ lipopolysaccharide acute toxic lethal hepatitis. Hepatology. Mar 2002;35(3):535-543.
3. Sharabi K, Tavares CD, Rines AK, Puigserver P. Molecular pathophysiology of hepatic glucose production. Mol Aspects Med. Dec 2015;46:21-33.
4. Yoon JC, Puigserver P, Chen G, et al. Control of hepatic gluconeogenesis through the transcriptional coactivator PGC-1. Nature. Sep 13 2001;413(6852):131-138.
5. Puigserver P, Rhee J, Donovan J, et al. Insulin-regulated hepatic gluconeogenesis through FOXO1-PGC-1alpha interaction. Nature. May 29 2003;423(6939):550-555.
6. Cheng Z, Guo S, Copps K, et al. Foxo1 integrates insulin signaling with mitochondrial function in the liver. Nat Med. Nov 2009;15(11):1307-1311.
7. Rhee J, Inoue Y, Yoon JC, et al. Regulation of hepatic fasting response by PPARgamma coactivator-1alpha (PGC-1): requirement for hepatocyte nuclear factor 4alpha in gluconeogenesis. Proc Natl Acad Sci U S A. Apr 1 2003;100(7):4012-4017.
8. Zheng J, Woo SL, Hu X, et al. Metformin and metabolic diseases: a focus on hepatic aspects. Front Med. Jun 2015;9(2):173-186.
9. Finck BN, Kelly DP. PGC-1 coactivators: inducible regulators of energy metabolism in health and disease. J Clin Invest. 2006;116(3):615-622.
10. Michael LF, Wu Z, Cheatham RB, et al. Restoration of insulin-sensitive glucose transporter (GLUT4) gene expression in muscle cells by the transcriptional coactivator PGC-1. Proc Natl Acad Sci U S A. Mar 27 2001;98(7):3820-3825.
11. Wallberg-Henriksson H, Zierath JR. GLUT4: a key player regulating glucose homeostasis? Insights from transgenic and knockout mice (review). Mol Membr Biol. Jul-Sep 2001;18(3):205-211.
12. Thurmond DC, Pessin JE. Molecular machinery involved in the insulin-regulated fusion of GLUT4-containing vesicles with the plasma membrane (review). Mol Membr Biol. Oct-Dec 2001;18(4):237-245.
13. Martin S, Slot JW, James DE. GLUT4 trafficking in insulin-sensitive cells. A morphological review. Cell Biochem Biophys. 1999;30(1):89-113.
14. Zorzano A, Sevilla L, Tomas E, Camps M, Guma A, Palacin M. Trafficking pathway of GLUT4 glucose transporters in muscle (review). Int J Mol Med. Sep 1998;2(3):263-271.
15. Flower D. The lipocalin protein family: structure and function. Biochem J. 1996;318:1-14.
16. Schlehuber S, Skerra A. Lipocalins in drug discovery: from natural ligand-binding proteins to "anticalins". Drug Discov Today. 2005;10(1):23-33.
17. Suh JB, Kim SM, Cho GJ, Choi KM, Han JH, Taek Geun H. Elevated serum retinol-binding protein 4 is associated with insulin resistance in older women. Metabolism. Jan 2010;59(1):118-122.
18. Choi KM, Kim TN, Yoo HJ, et al. Effect of exercise training on A-FABP, lipocalin-2 and RBP4 levels in obese women. Clin Endocrinol (Oxf). Apr 2009;70(4):569-574.
19. Fernandez-Real JM, Moreno JM, Ricart W. Circulating retinol-binding protein-4 concentration might reflect insulin resistance-associated iron overload. Diabetes. Jul 2008;57(7):1918-1925.
20. Zhou Y, Rui L. Lipocalin 13 regulation of glucose and lipid metabolism in obesity. Vitam Horm. 2013;91:369-383.
21. Asokan A, Schaffer DV, Samulski RJ. The AAV vector toolkit: poised at the clinical crossroads. Mol Ther. Apr 2012;20(4):699-708.
22. Bell P, Gao G, Haskins ME, et al. Evaluation of adeno-associated viral vectors for liver-directed gene transfer in dogs. Hum Gene Ther. Aug 2011;22(8):985-997.
23. Grimm D, Kay MA. From virus evolution to vector revolution: use of naturally occurring serotypes of adeno-associated virus (AAV) as novel vectors for human gene therapy. Curr Gene Ther. Aug 2003;3(4):281-304.
24. Thomas CE, Storm TA, Huang Z, Kay MA. Rapid uncoating of vector genomes is the key to efficient liver transduction with pseudotyped adeno-associated virus vectors. J Virol. Mar 2004;78(6):3110-3122.
25. Gao G, Lu Y, Calcedo R, et al. Biology of AAV serotype vectors in liver-directed gene transfer to nonhuman primates. Mol Ther. Jan 2006;13(1):77-87.
26. Cunningham SC, Dane AP, Spinoulas A, Logan GJ, Alexander IE. Gene delivery to the juvenile mouse liver using AAV2/8 vectors. Mol Ther. Jun 2008;16(6):1081-1088.
27. Xu H, Zhang L, Gu L, et al. Subretinal delivery of AAV2-mediated human erythropoietin gene is protective and safe in experimental diabetic retinopathy. Invest Ophthalmol Vis Sci. Mar 2014;55(3):1519-1530.
28. Hajjar RJ, Zsebo K, Deckelbaum L, et al. Design of a phase 1/2 trial of intracoronary administration of AAV1/SERCA2a in patients with heart failure. J Card Fail. Jun 2008;14(5):355-367.
29. Nathwani AC, Rosales C, McIntosh J, et al. Long-term safety and efficacy following systemic administration of a self-complementary AAV vector encoding human FIX pseudotyped with serotype 5 and 8 capsid proteins. Mol Ther. May 2011;19(5):876-885.
30. Nathwani AC, Tuddenham EG, Rangarajan S, et al. Adenovirus-associated virus vector-mediated gene transfer in hemophilia B. N Engl J Med. Dec 22 2011;365(25):2357-2365.
31. Vandenberghe LH, Wilson JM, Gao G. Tailoring the AAV vector capsid for gene therapy. Gene Ther. Mar 2009;16(3):311-319.
32. Lagor WR, Johnston JC, Lock M, Vandenberghe LH, Rader DJ. Adeno-associated viruses as liver-directed gene delivery vehicles: focus on lipoprotein metabolism. Methods in molecular biology. 2013;1027:273-307.
33. Zangi L, Lui KO, von Gise A, et al. Modified mRNA directs the fate of heart progenitor cells and induces vascular regeneration after myocardial infarction. Nat Biotechnol. Oct 2013;31(10):898-907.
34. Kormann MS, Hasenpusch G, Aneja MK, et al. Expression of therapeutic proteins after delivery of chemically modified mRNA in mice. Nat Biotechnol. Feb 2011;29(2):154-157.
35. McIvor RS. Therapeutic delivery of mRNA: the medium is the message. Mol Ther. May 2011;19(5):822-823.
36. Zhu H, Yu L, He Y, Wang B. Nonhuman primate models of type 1 diabetes mellitus for islet transplantation. J Diabetes Res. 2014;2014:785948.
37. He S, Wang D, Wei L. Practical and critical instruction for nonhuman primate diabetic models. Transplant Proc. Jun 2013;45(5):1856-1865.
38. Bluestone JA, Herold K, Eisenbarth G. Genetics, pathogenesis and clinical interventions in type 1 diabetes. Nature. Apr 29 2010;464(7293):1293-1300.
39. Wagner JD, Cline JM, Shadoan MK, Bullock BC, Rankin SE, Cefalu WT. Naturally occurring and experimental diabetes in cynomolgus monkeys: a comparison of carbohydrate and lipid metabolism and islet pathology. Toxicol Pathol. Jan-Feb 2001;29(1):142-148.
40. Livingstone SJ, Levin D, Looker HC, et al. Estimated life expectancy in a Scottish cohort with type 1 diabetes, 2008-2010. Jama. Jan 6 2015;313(1):37-44.
41. Kowalski AJ. Can we really close the loop and how soon? Accelerating the availability of an artificial pancreas: a roadmap to better diabetes outcomes. Diabetes Technol Ther. Jun 2009;11 Suppl 1:S113-119.
42. Heinemann L, Benesch C, DeVries JH. AP@home: The Artificial Pancreas Is Now at Home. J Diabetes Sci Technol. Feb 16 2016.
43. Jacobs PG, El Youssef J, Castle JR, et al. Development of a fully automated closed loop artificial pancreas control system with dual pump delivery of insulin and glucagon. Conf Proc IEEE Eng Med Biol Soc. 2011;2011:397-400.
44. Dassau E, Atlas E, Phillip M. Closing the loop. Int J Clin Pract Suppl. Feb 2010(166):20-25.
45. Steil G, Rebrin K, Mastrototaro JJ. Metabolic modelling and the closed-loop insulin delivery problem. Diabetes Res Clin Pract. Dec 2006;74 Suppl 2:S183-186.
46. Jamiolkowski RM, Guo LY, Li YR, Shaffer SM, Naji A. Islet transplantation in type I diabetes mellitus. Yale J Biol Med. Mar 2012;85(1):37-43.
47. Shapiro AM, Ricordi C, Hering BJ, et al. International trial of the Edmonton protocol for islet transplantation. N Engl J Med. Sep 28 2006;355(13):1318-1330.
48. Giannoukakis N, Trucco M. Cellular therapies based on stem cells and their insulin-producing surrogates: a 2015 reality check. Pediatr Diabetes. 2015;16(3):151-163.
49. Tahrani AA, Barnett AH, Bailey CJ. SGLT inhibitors in management of diabetes. Lancet Diabetes Endocrinol. Oct 2013;1(2):140-151.
50. Trujillo JM, Nuffer W, Ellis SL. GLP-1 receptor agonists: a review of head-to-head clinical studies. Ther Adv Endocrinol Metab. Feb 2015;6(1):19-28.
51. Tella SH, Rendell MS. Glucagon-like polypeptide agonists in type 2 diabetes mellitus: efficacy and tolerability, a balance. Ther Adv Endocrinol Metab. Jun 2015;6(3):109-134.
52. Bode BW, Garg SK. The Emerging Role of Adjunctive Noninsulin Antihyperglycemic Therapy in the Management of Type 1 Diabetes. Endocr Pract. Feb 2016;22(2):220-230.
53. Novikov A, Vallon V. Sodium glucose cotransporter 2 inhibition in the diabetic kidney: an update. Curr Opin Nephrol Hypertens. Jan 2016;25(1):50-58.
54. Tomlinson B, Hu M, Zhang Y, Chan P, Liu ZM. An overview of new GLP-1 receptor agonists for type 2 diabetes. Expert Opin Investig Drugs. Feb 2016;25(2):145-158.
55. Njomnang Soh P, Vidal F, Huyghe E, Gourdy P, Halimi JM, Bouhanick B. Urinary and genital infections in patients with diabetes: How to diagnose and how to treat. Diabetes Metab. Feb 2016;42(1):16-24.
56. Taylor SI, Blau JE, Rother KI. SGLT2 Inhibitors May Predispose to Ketoacidosis. J Clin Endocrinol Metab. Aug 2015;100(8):2849-2852.
57. Syed SH, Gosavi S, Shami W, et al. A Review of Sodium Glucose Co-transporter 2 Inhibitors Canagliflozin, Dapagliflozin and Empagliflozin. Cardiovasc Hematol Agents Med Chem.2015;13(2):105-112.
58. Scheen AJ. SGLT2 inhibition: efficacy and safety in type 2 diabetes treatment. Expert Opin Drug Saf. Dec 2015;14(12):1879-1904.
59. Rosenstock J, Ferrannini E. Euglycemic Diabetic Ketoacidosis: A Predictable, Detectable, and Preventable Safety Concern With SGLT2 Inhibitors. Diabetes Care. Sep 2015;38(9):1638-1642.
60. Garber AJ. Long-acting glucagon-like peptide 1 receptor agonists: a review of their efficacy and tolerability. Diabetes Care. May 2011;34 Suppl 2:S279-284.

## Claims

1. An effective amount of A20 for use in treating a condition selected from the group consisting of hyperglycemia, diabetes, pre-diabetes, insulin resistance and metabolic syndrome, wherein the A20 is delivered to the liver.

2. The effective amount of A20 for use according to claim 1, wherein the diabetes is insulin-dependent diabetes (Type 1 diabetes), Type 2 diabetes, or gestational diabetes.

3. The effective amount of A20 for use according to claim 1 or 2, wherein the A20 is to be administered as A20 protein or as A20 gene therapy.

4. An agent that upregulates A20 expression for use in treating a condition selected from the group consisting of hyperglycemia, diabetes, pre-diabetes, insulin resistance and metabolic syndrome, wherein the agent is delivered to and upregulates A20 expression in one or more tissues in the subject in an effective amount to treat the condition;
(i) wherein the agent comprises a nucleic acid encoding a gene for A20 in an expression system; or
(ii) wherein the agent increases the expression of endogenous A20 in the subject:
(a) by activating one or more endogenous promoters of A20; or
(b) by editing the genome of the subject, wherein editing the genome of the subject comprises inserting one or more exogenous promoters; and
wherein the tissue is liver.

5. The agent for use according to claim 4, wherein the diabetes is insulin-dependent diabetes (Type 1 diabetes), Type 2 diabetes, or gestational diabetes.

6. The agent for use according to claim 4, wherein the expression system comprises one or more promoters.

7. The agent for use according to any one of claims 4-6, wherein administering the agent restores euglycemia in the subject.

8. An agent that upregulates A20 in the liver of a subject for use in treating insulin-dependent diabetes mellitus; wherein the agent comprises an expression system:
(i) wherein the expression system comprises one or more promoters; and/or
(ii) wherein the expression system further comprises a nucleic acid encoding A20, and wherein the agent is delivered to liver.

9. The agent for use according to any one of claims 4-8, wherein the expression system is delivered by a viral vector.

10. The agent for use according to claim 9 wherein the viral vector comprises a recombinant AAV vector.

11. The agent for use according to claim 10:
(i) wherein the AAV vector comprises a genome derived from AAV serotype AAV2; and/or
(ii) wherein the AAV vector is modified to comprise a capsid with tropism for tissue in the liver.

12. The agent for use according to claim 11, wherein the AAV vector comprises a capsid protein that is derived from AAV serotype AAV8.

13. The effective amount of A20 for use according to any one of claims 1-3, wherein administering A20 restores euglycemia in the subject.

14. The agent for use according to any one of claims 8-12, wherein administering the agent restores euglycemia in the subject.

## Patentansprüche

1. Eine wirksame Menge von A20 zur Verwendung bei der Behandlung eines Zustands, ausgewählt aus der Gruppe bestehend aus Hyperglykämie, Diabetes, Prä-Diabetes, Insulinresistenz und metabolischem Syndrom, wobei das A20 der Leber zugeführt wird.

2. Die wirksame Menge von A20 zur Verwendung gemäß Anspruch 1, wobei der Diabetes ein insulinabhängiger Diabetes (Typ-1-Diabetes), Typ-2-Diabetes oder Gestationsdiabetes ist.

3. Die wirksame Menge von A20 zur Verwendung gemäß Anspruch 1 oder 2, wobei das A20 als A20-Protein oder als A20-Gentherapie verabreicht wird.

4. Ein Agens, das die A20-Expression hochreguliert, zur Verwendung bei der Behandlung eines Zustands, ausgewählt aus der Gruppe bestehend aus Hyperglykämie, Diabetes, Prädiabetes, Insulinresistenz und metabolischem Syndrom, wobei das Agens an ein oder mehrere Gewebe des Patienten zugeführt wird und dort die A20-Expression hochreguliert oder mehreren Geweben in dem Subjekt in einer wirksamen Menge zur Behandlung des Zustands hochreguliert;
(i) wobei das Agens eine Nukleinsäure umfasst, die für ein Gen für A20 in einem Expressionssystem kodiert; oder
(ii) wobei das Agens die Expression von endogenem A20 in dem Subjekt erhöht:
(a) durch Aktivieren eines Promoters oder mehrerer endogener Promotoren von A20; oder
(b) durch Editieren des Genoms des Subjekts, wobei das Editieren des Genoms des Subjekts das Einfügen eines oder mehrerer exogener Promotoren umfasst; und wobei das Gewebe Leber ist.

5. Das Agens zur Verwendung gemäß Anspruch 4, wobei der Diabetes ein insulinabhängiger Diabetes (Typ 1 Diabetes), Typ 2 Diabetes oder Gestationsdiabetes ist.

6. Das Agens zur Verwendung gemäß Anspruch 4, wobei das Expressionssystem einen oder mehrere Promotoren umfasst.

7. Das Agens zur Verwendung gemäß einem der Ansprüche 4-6, wobei die Verabreichung des Agens Euglykämie in dem Subjekt wiederherstellt.

8. Ein Agens, das A20 in der Leber eines Patienten hochreguliert, zur Verwendung bei der Behandlung von insulinabhängigem Diabetes mellitus; wobei das Agens ein Expressionssystem umfasst:
(i) wobei das Expressionssystem einen oder mehrere Promotoren umfasst; und/oder
(ii) wobei das Expressionssystem ferner eine Nukleinsäure umfasst, die für A20 kodiert, und wobei das Agens der Leber zugeführt wird.

9. Das Agens zur Verwendung gemäß einem der Ansprüche 4-8, wobei das Expressionssystem durch einen viralen Vektor zugeführt wird.

10. Das Agens zur Verwendung gemäß Anspruch 9, wobei der virale Vektor einen rekombinanten AAV-Vektor umfasst.

11. Das Agens zur Verwendung gemäß Anspruch 10:
(i) wobei der AAV-Vektor ein Genom umfasst, das vom AAV-Serotyp AAV2 abgeleitet ist; und/oder
(ii) wobei der AAV-Vektor so modifiziert ist, dass er ein Kapsid mit Tropismus für Gewebe in der Leber umfasst.

12. Das Agens zur Verwendung gemäß Anspruch 11, wobei der AAV-Vektor ein Kapsid Protein umfasst, das vom AAV-Serotyp AAV8 abgeleitet ist.

13. Die wirksame Menge von A20 zur Verwendung gemäß einem der Ansprüche 1-3, wobei die Verabreichung von A20 Euglykämie in dem Patienten wiederherstellt.

14. Das Agens zur Verwendung gemäß einem der Ansprüche 8-12, wobei die Verabreichung des Agens Euglykämie in dem Patienten wiederherstellt.

## Revendications

1. - Quantité efficace de A20 pour une utilisation dans le traitement d'un état choisi dans le groupe consistant en l'hyperglycémie, le diabète, le pré-diabète, l'insulino-résistance et le syndrome métabolique, dans laquelle le A20 est délivré au foie.

2. - Quantité efficace de A20 pour l'utilisation selon la revendication 1, dans laquelle le diabète est un diabète insulino-dépendant (diabète de type 1), un diabète de type 2, ou un diabète gestationnel.

3. - Quantité efficace de A20 pour l'utiliation selon l'une des revendications 1 ou 2, dans laquelle le A20 doit être administré sous forme de protéine A20 ou de thérapie génique A20.

4. - Agent qui régule à la hausse l'expression de A20 pour une utilisation dans le traitement d'un état choisi dans le groupe consistant en l'hyperglycémie, le diabète, le pré-diabète, l'insulino-résistance et le syndrome métabolique, dans lequel l'agent est délivré à et régule à la hausse l'expression de A20 dans un ou plusieurs tissus du sujet en une quantité efficace pour traiter l'état ;
(i) dans lequel l'agent comprend un acide nucléique codant pour un gène pour A20 dans un système d'expression ; ou
(ii) dans lequel l'agent augmente l'expression de A20 endogène chez le sujet :
(a) en activant un ou plusieurs promoteurs endogènes de A20 ; ou
(b) en éditant le génome du sujet, dans lequel l'édition du génome du sujet comprend l'insertion d'un ou plusieurs promoteurs exogènes ; et
dans lequel le tissu est le foie.

5. - Agent pour l'utilisation selon la revendication 4, dans lequel le diabète est un diabète insulino-dépendant (diabète de type 1), un diabète de type 2 ou un diabète gestationnel.

6. - Agent pour l'utilisation selon la revendication 4, dans lequel le système d'expression comprend un ou plusieurs promoteurs.

7. - Agent pour l'utilisation selon l'une quelconque des revendications 4 à 6, dans lequel l'administration de l'agent rétablit l'euglycémie chez le sujet.

8. - Agent qui régule à la hausse A20 dans le foie d'un sujet pour une utilisation dans le traitement du diabète sucré insulino-dépendant ; dans lequel l'agent comprend un système d'expression :
(i) dans lequel le système d'expression comprend un ou plusieurs promoteurs ; et/ou
(ii) dans lequel le système d'expression comprend en outre un acide nucléique codant pour A20, et dans lequel l'agent est délivré au foie.

9. - Agent pour l'utilisation selon l'une quelconque des revendications 4 à 8, dans lequel le système d'expression est délivré par un vecteur viral.

10. - Agent pour l'utilisation selon la revendication 9, dans lequel le vecteur viral comprend un vecteur AAV recombinant.

11. - Agent pour l'utilisation selon la revendication 10 :
(i) dans lequel le vecteur AAV comprend un génome dérivé du sérotype d'AAV AAV2 ; et/ou
(ii) dans lequel le vecteur AAV est modifié pour comprendre une capside ayant un tropisme pour les tissus du foie.

12. - Agent pour l'utilisation selon la revendication 11, dans lequel le vecteur AAV comprend une protéine de capside qui est dérivée du sérotype d'AAV AAV8.

13. - Quantité efficace de A20 pour l'utilisation selon l'une quelconque des revendications 1 à 3, dans laquelle l'administration de A20 rétablit l'euglycémie chez le sujet.

14. - Agent pour l'utilisation selon l'une quelconque des revendications 8 à 12, dans lequel l'administration de l'agent rétablit l'euglycémie chez le sujet.


### SEQUENCE LISTING

<110> Beth Israel Deaconess Medical Center, Inc.
<120> NOVEL THERAPY TO ACHIEVE GLYCEMIC CONTROL
<130> B0662.70095WO00
<140> PCT/US2017/046938
   <141> 2017-08-15
<150> US 62/375,427
   <151> 2016-08-15
<150> US 62/382,726
   <151> 2016-09-01
<160> 4
<170> PatentIn version 3.5
<210> 1
   <211> 2373
   <212> DNA
   <213> Homo sapiens
<400> 1
   atggctgaac aagtccttcc tcaggctttg tatttgagca atatgcggaa agctgtgaag      60
   atacgggaga gaactccaga agacattttt aaacctacta atgggatcat tcatcatttt     120
   aaaaccatgc accgatacac actggaaatg ttcagaactt gccagttttg tcctcagttt     180
   cgggagatca tccacaaagc cctcatcgac agaaacatcc aggccaccct ggaaagccag     240
   aagaaactca actggtgtcg agaagtccgg aagcttgtgg cgctgaaaac gaacggtgac     300
   ggcaattgcc tcatgcatgc cacttctcag tacatgtggg gcgttcagga cacagacttg     360
   gtactgagga aggcgctgtt cagcacgctc aaggaaacag acacacgcaa ctttaaattc     420
   cgctggcaac tggagtctct caaatctcag gaatttgttg aaacggggct ttgctatgat     480
   actcggaact ggaatgatga atgggacaat cttatcaaaa tggcttccac agacacaccc     540
   atggcccgaa gtggacttca gtacaactca ctggaagaaa tacacatatt tgtcctttgc     600
   aacatcctca gaaggccaat cattgtcatt tcagacaaaa tgctaagaag tttggaatca     660
   ggttccaatt tcgccccttt gaaagtgggt ggaatttact tgcctctcca ctggcctgcc     720
   caggaatgct acagataccc cattgttctc ggctatgaca gccatcattt tgtacccttg     780
   gtgaccctga aggacagtgg gcctgaaatc cgagctgttc cacttgttaa cagagaccgg     840
   ggaagatttg aagacttaaa agttcacttt ttgacagatc ctgaaaatga gatgaaggag     900
   aagctcttaa aagagtactt aatggtgata gaaatccccg tccaaggctg ggaccatggc     960
   acaactcatc tcatcaatgc cgcaaagttg gatgaagcta acttaccaaa agaaatcaat    1020
   ctggtagatg attactttga acttgttcag catgagtaca agaaatggca ggaaaacagc    1080
   gagcagggga ggagagaggg gcacgcccag aatcccatgg aaccttccgt gccccagctt 1140
   tctctcatgg atgtaaaatg tgaaacgccc aactgcccct tcttcatgtc tgtgaacacc 1200
   cagcctttat gccatgagtg ctcagagagg cggcaaaaga atcaaaacaa actcccaaag 1260
   ctgaactcca agccgggccc tgaggggctc cctggcatgg cgctcggggc ctctcgggga 1320
   gaagcctatg agcccttggc gtggaaccct gaggagtcca ctggggggcc tcattcggcc 1380
   ccaccgacag cacccagccc ttttctgttc agtgagacca ctgccatgaa gtgcaggagc 1440
   cccggctgcc ccttcacact gaatgtgcag cacaacggat tttgtgaacg ttgccacaac 1500
   gcccggcaac ttcacgccag ccacgcccca gaccacacaa ggcacttgga tcccgggaag 1560
   tgccaagcct gcctccagga tgttaccagg acatttaatg ggatctgcag tacttgcttc 1620
   aaaaggacta cagcagaggc ctcctccagc ctcagcacca gcctccctcc ttcctgtcac 1680
   cagcgttcca agtcagatcc ctcgcggctc gtccggagcc cctccccgca ttcttgccac 1740
   agagctggaa acgacgcccc tgctggctgc ctgtctcaag ctgcacggac tcctggggac 1800
   aggacgggga cgagcaagtg cagaaaagcc ggctgcgtgt attttgggac tccagaaaac 1860
   aagggctttt gcacactgtg tttcatcgag tacagagaaa acaaacattt tgctgctgcc 1920
   tcagggaaag tcagtcccac agcgtccagg ttccagaaca ccattccgtg cctggggagg 1980
   gaatgcggca cccttggaag caccatgttt gaaggatact gccagaagtg tttcattgaa 2040
   gctcagaatc agagatttca tgaggccaaa aggacagaag agcaactgag atcgagccag 2100
   cgcagagatg tgcctcgaac cacacaaagc acctcaaggc ccaagtgcgc ccgggcctcc 2160
   tgcaagaaca tcctggcctg ccgcagcgag gagctctgca tggagtgtca gcatcccaac 2220
   cagaggatgg gccctggggc ccaccggggt gagcctgccc ccgaagaccc ccccaagcag 2280
   cgttgccggg cccccgcctg tgatcatttt ggcaatgcca agtgcaacgg ctactgcaac 2340
   gaatgctttc agttcaagca gatgtatggc taa 2373
<210> 2
   <211> 790
   <212> PRT
   <213> Homo sapiens
<400> 2
<210> 3
   <211> 2328
   <212> DNA
   <213> Mus musculus
<400> 3
   atggctgaac aacttcttcc tcaggctttg tatttgagca atatgcggaa agctgtgaag 60
   atacgagaga gaaccccaga agacattttc aaacctacca atgggatcat ctatcacttt 120
   aaaaccatgc accgatacac gctggagatg ttcagaacat gccagttttg cccacagttc 180
   cgagagatca tccacaaagc acttattgac agaagtgtcc aggcttccct ggaaagccag 240
   aagaagctca actggtgtcg tgaagtcagg aagctcgtgg ctctgaaaac caatggtgat 300
   ggaaactgcc tcatgcatgc agcttgtcag tacatgtggg gtgttcagga tactgacctg 360
   gtcctgagga aggccctctg cagcaccctt aaggagacag acactcggaa ctttaaattc 420
   cgctggcagc tggaatctct gaaatctcag gaatttgtgg aaacaggact ttgctacgac 480
   actcggaact ggaatgacga atgggacaac ttggtcaaaa tggcatcagc agacacacct 540
   gcagcccgaa gtggacttca gtacaattcc ctggaagaaa tccacatatt tgtcctcagc 600
   aacatcctca gaagacccat cattgtcatt tcagacaaaa tgctaagaag tttggaatct 660
   ggttccaatt ttgctccttt gaaagtgggt gggatttatc tgcctcttca ctggcctgcc 720
   caggagtgtt acagatatcc catcgtccta ggctatgaca gccagcactt tgtacccctg 780
   gtgaccctga aggacagtgg acctgaactt cgcgctgttc cacttgttaa cagagaccgg 840
   ggtaggtttg aagacttaaa agttcacttc ttgacagatc ctgagaatga gatgaaggaa 900
   aagcttctaa aggagtactt gatagtgatg gagatccctg tgcaaggctg ggaccacggc 960
   acgactcacc tgatcaacgc tgcaaaattg gatgaagcta acttacccaa agaaataaat 1020
   ttggtagacg attactttga gcttgttcag cacgaataca agaaatggca ggagaacagc 1080
   gatcaggcca ggagagcggc acatgcgcag aaccccttgg agccttccac accccagcta 1140
   tcactcatgg atataaaatg tgagacaccc aactgtcctt tcttcatgtc cgtgaacact 1200
   cagcctttat gccacgaatg ctcagagagg cgccaaaaga atcagagcaa gctcccaaag 1260
   ctgaactcga agctaggccc tgaaggactc ccaggcgtgg gacttggctc ctcaaactgg 1320
   agccccgagg aaaccgctgg aggacctcat tcagccccac ccacagcacc cagccttttt 1380
   ctcttcagtg agaccactgc aatgaagtgc aggagtcctg ggtgcccttt tactttgaat 1440
   gtgcagcata atggattctg tgagcgttgc cacgcccggc agattaatgc cagccacacc 1500
   gcagaccctg gaaagtgcca agcctgcctt caggatgtca ctcggacctt taatggcatc 1560
   tgcagtacct gtttcaaaag gactacagca gagcccagct ccagcctcac ttccagtatc 1620
   cctgcctcct gtcaccaacg ctccaagtct gacccctcac aactcatcca aagtctcact 1680
   ccacactctt gccaccggac tggaaatgtc tctccttctg gctgcctctc ccaggctgca 1740
   cggactccag gagacagagc agggacaagc aagtgcagga aagctggctg catgtatttt 1800
   gggactccag aaaacaaggg cttttgcact ctatgtttca tcgaatacag agaaaataag 1860
   cagtctgtta ctgcctctga gaaagctggt tccccggccc ccaggttcca gaacaatgtc 1920
   ccgtgcctgg gcagggagtg cggcacactc ggaagcacca tgtttgaagg gtactgtcag 1980
   aagtgtttca tcgaagctca gaaccagaga ttccatgaag caagaagaac ggaagaacag 2040
   ctgagatcaa gccagcatag agacatgcct cgaactacac aggtagcctc aaggctgaaa 2100
   tgtgcccggg cctcctgcaa gaacattctg gcctgtcgca gtgaggaact ctgtatggag 2160
   tgccagcacc taagccaacg agtaggttct gtggcccacc ggggtgagcc cacgcctgaa 2220
   gagcccccta aacagcgctg ccgggcccct gcttgtgatc actttggcaa tgccaagtgt 2280
   aatggttact gcaatgagtg ctaccagttc aagcagatgt atggctaa 2328
<210> 4
   <211> 775
   <212> PRT
   <213> Mus musculus
<400> 4
   Met Ala Glu Gln Leu Leu Pro Gln Ala Leu Tyr Leu Ser Asn Met Arg
   1 5 10 15
   Lys Ala Val Lys Ile Arg Glu Arg Thr Pro Glu Asp Ile Phe Lys Pro
   20 25 30
   Thr Asn Gly Ile Ile Tyr His Phe Lys Thr Met His Arg Tyr Thr Leu
   35 40 45
   Glu Met Phe Arg Thr Cys Gln Phe Cys Pro Gln Phe Arg Glu Ile Ile
   50 55 60
   His Lys Ala Leu Ile Asp Arg Ser Val Gln Ala Ser Leu Glu Ser Gln
   65 70 75 80
   Lys Lys Leu Asn Trp Cys Arg Glu Val Arg Lys Leu Val Ala Leu Lys
   85 90 95
   Thr Asn Gly Asp Gly Asn Cys Leu Met His Ala Ala Cys Gln Tyr Met
   100 105 110
   Trp Gly Val Gln Asp Thr Asp Leu Val Leu Arg Lys Ala Leu Cys Ser
   115 120 125
   Thr Leu Lys Glu Thr Asp Thr Arg Asn Phe Lys Phe Arg Trp Gln Leu
   130 135 140
   Glu Ser Leu Lys Ser Gln Glu Phe Val Glu Thr Gly Leu Cys Tyr Asp
   145                 150                 155                 160
   Thr Arg Asn Trp Asn Asp Glu Trp Asp Asn Leu Val Lys Met Ala Ser
                   165                 170                 175
   Ala Asp Thr Pro Ala Ala Arg Ser Gly Leu Gln Tyr Asn Ser Leu Glu
               180                 185                 190
   Glu Ile His Ile Phe Val Leu Ser Asn Ile Leu Arg Arg Pro Ile Ile
           195                 200                 205
   Val Ile Ser Asp Lys Met Leu Arg Ser Leu Glu Ser Gly Ser Asn Phe
       210                 215                 220
   Ala Pro Leu Lys Val Gly Gly Ile Tyr Leu Pro Leu His Trp Pro Ala
   225                 230                 235                 240
   Gln Glu Cys Tyr Arg Tyr Pro Ile Val Leu Gly Tyr Asp Ser Gln His
                   245                 250                 255
   Phe Val Pro Leu Val Thr Leu Lys Asp Ser Gly Pro Glu Leu Arg Ala
               260                 265                 270
   Val Pro Leu Val Asn Arg Asp Arg Gly Arg Phe Glu Asp Leu Lys Val
           275                 280                 285
   His Phe Leu Thr Asp Pro Glu Asn Glu Met Lys Glu Lys Leu Leu Lys
       290                 295                 300
   Glu Tyr Leu Ile Val Met Glu Ile Pro Val Gln Gly Trp Asp His Gly
   305                 310                 315                 320
   Thr Thr His Leu Ile Asn Ala Ala Lys Leu Asp Glu Ala Asn Leu Pro
                   325                 330                 335
   Lys Glu Ile Asn Leu Val Asp Asp Tyr Phe Glu Leu Val Gln His Glu
               340                 345                 350
   Tyr Lys Lys Trp Gln Glu Asn Ser Asp Gln Ala Arg Arg Ala Ala His
           355                 360                 365
   Ala Gln Asn Pro Leu Glu Pro Ser Thr Pro Gln Leu Ser Leu Met Asp
       370                 375                 380
   Ile Lys Cys Glu Thr Pro Asn Cys Pro Phe Phe Met Ser Val Asn Thr
   385 390 395 400
   Gln Pro Leu Cys His Glu Cys Ser Glu Arg Arg Gln Lys Asn Gln Ser
   405 410 415
   Lys Leu Pro Lys Leu Asn Ser Lys Leu Gly Pro Glu Gly Leu Pro Gly
   420 425 430
   Val Gly Leu Gly Ser Ser Asn Trp Ser Pro Glu Glu Thr Ala Gly Gly
   435 440 445
   Pro His Ser Ala Pro Pro Thr Ala Pro Ser Leu Phe Leu Phe Ser Glu
   450 455 460
   Thr Thr Ala Met Lys Cys Arg Ser Pro Gly Cys Pro Phe Thr Leu Asn
   465 470 475 480
   Val Gln His Asn Gly Phe Cys Glu Arg Cys His Ala Arg Gln Ile Asn
   485 490 495
   Ala Ser His Thr Ala Asp Pro Gly Lys Cys Gln Ala Cys Leu Gln Asp
   500 505 510
   Val Thr Arg Thr Phe Asn Gly Ile Cys Ser Thr Cys Phe Lys Arg Thr
   515 520 525
   Thr Ala Glu Pro Ser Ser Ser Leu Thr Ser Ser Ile Pro Ala Ser Cys
   530 535 540
   His Gln Arg Ser Lys Ser Asp Pro Ser Gln Leu Ile Gln Ser Leu Thr
   545 550 555 560
   Pro His Ser Cys His Arg Thr Gly Asn Val Ser Pro Ser Gly Cys Leu
   565 570 575
   Ser Gln Ala Ala Arg Thr Pro Gly Asp Arg Ala Gly Thr Ser Lys Cys
   580 585 590
   Arg Lys Ala Gly Cys Met Tyr Phe Gly Thr Pro Glu Asn Lys Gly Phe
   595 600 605
   Cys Thr Leu Cys Phe Ile Glu Tyr Arg Glu Asn Lys Gln Ser Val Thr
   610 615 620
   Ala Ser Glu Lys Ala Gly Ser Pro Ala Pro Arg Phe Gln Asn Asn Val
   625 630 635 640
   Pro Cys Leu Gly Arg Glu Cys Gly Thr Leu Gly Ser Thr Met Phe Glu
                   645                 650                 655
   Gly Tyr Cys Gln Lys Cys Phe Ile Glu Ala Gln Asn Gln Arg Phe His
               660                 665                 670
   Glu Ala Arg Arg Thr Glu Glu Gln Leu Arg Ser Ser Gln His Arg Asp
           675                 680                 685
   Met Pro Arg Thr Thr Gln Val Ala Ser Arg Leu Lys Cys Ala Arg Ala
       690                 695                 700
   Ser Cys Lys Asn Ile Leu Ala Cys Arg Ser Glu Glu Leu Cys Met Glu
   705                 710                 715                 720
   Cys Gln His Leu Ser Gln Arg Val Gly Ser Val Ala His Arg Gly Glu
                   725                 730                 735
   Pro Thr Pro Glu Glu Pro Pro Lys Gln Arg Cys Arg Ala Pro Ala Cys
               740                 745                 750
   Asp His Phe Gly Asn Ala Lys Cys Asn Gly Tyr Cys Asn Glu Cys Tyr
           755                 760                 765
   Gln Phe Lys Gln Met Tyr Gly
       770                 775